**Europäisches Patentamt**

**European Patent Office**

**Office européen des brevets**

(19)

(11) Veröffentlichungsnummer: **0 236 294**

**B1**

(12) **EUROPÄISCHE PATENTSCHRIFT**

(45) Veröffentlichungstag der Patentschrift:
08.03.89

(21) Anmeldenummer: 85900467.3

(22) Anmeldetag: 29.12.84

(86) Internationale Anmeldenummer:
PCT/EP 84/00434

(87) Internationale Veröffentlichungsnummer:
WO 86/04141 (17.07.86 Gazette 86/17)

(51) Int. Cl.⁴: **G 01 N 25/14, G 01 N 33/483**

(54) VERFAHREN ZUR HERSTELLUNG UND VERGLEICH VON KRISTALLBILDERN.

(43) Veröffentlichungstag der Anmeldung:
16.09.87 Patentblatt 87/38

(45) Bekanntmachung des Hinweises auf die Patenterteilung:
08.03.89 Patentblatt 89/10

(84) Benannte Vertragsstaaten:
AT CH DE GB LI

(56) Entgegenhaltungen:
DE-C-825 906

Die Akte enthält technische Angaben, die nach
dem Eingang der Anmeldung eingereicht wurden
und die nicht in dieser Patentschrift enthalten sind.

(73) Patentinhaber: **HEINZ SPAGYRIK INSTITUT AG,
CH- Corticiasca (TI) (CH)**

(72) Erfinder: **Heinz, Ulrich Jürgen, Prof. Dr.,
Verdistrasse 11, I-39012 Meran (BZ) (IT)**

(74) Vertreter: **Jahn- Held, Wilhelm W. Dr.Dr.- Ing.
Dipl.- Chem., Schöne Aussicht 8, D-3513
Staufenberg- Landwehrhagen (DE)**

## Beschreibung

R. Wernick, Das Beste aus Readers Digest, September 1984, Seite 27 - 31, trifft in seiner Veröffentlichung über "Das Blut, Spiegel unserer Geschichte", folgende Aufgabenstellung: "Vielleicht wird es den Ärzten der Zukunft dank den in einem einzigen Blutstropfen enthaltenen Informationen einmal gelingen, jedem Kranken eine massgeschneiderte Therapie zu verordnen".

Diese Aufgabenstellung haben A. und O. Selawry, Gustav Fischer Verlag, Stuttgart, 1957, in "Die Kupferchlorid - Kristallisation", noch nicht erkannt, obwohl dafür ein Bedürfnis der Wissenschaft und der Medizin besteht. Diese Autoren bechreiben die Beeinflussung von Salzen des morphologischen Typ NaCl, $CuCl_2$, und von anderen Salzen durch Zusätze von Salzen, Säuren, Laugen, pflanzlichen und animalischen Substanzen. Es wird die spezifische Empfindlichkeit auf die Kristallisation dieser Salze, insbesondere auf die Kristallisation von $CuCl_2$, ermittelt, und es werden die Veränderungen der Färbung und der Texturen beschrieben und dabei Formen der Aggregation mit Ähnlichkeitsbereichen festgestellt.

Es wird eine wässrige, 30-%-ige $CuCl_2$-Lösung mit einigen mg der Zusatzsubstanz auf einer Glasplatte versetzt und darüber eine Kristallisierschale gebildet. Es wird dann in einer Klimakammer bei 30°C und bei einer Luftfeuchtigkeit von 60 % 8 bis 10 Stunden die Flüssigkeit abgedunstet. Innerhalb von weiteren 2 Stunden erfolgt dann die Kristallisation des $CuCl_2$ unter Ausbildung charakteristischer Texturen der Kristallaggregate. Nach weiteren 2 Stunden Trocknungszeit wird die Glasplatte mit dem Kristallisat freigelegt und der makroskopischen Betrachtung unterzogen. Dazu wird ergänzemd eine mikroskopische Auswertung und eine photographische, Fixierung durchgeführt.

Die Reproduzierbarkeit dieser Versuche wird mit einer Streubreite von 20 % angegebem.

Es wird auch das System $CuCl_2$- Blut-Wasser umtersucht. Für Blut wird die Zusammensetzung 78 % Wasser; 21,56 % Eiweisstoffe; 0,44 % Kohlehydrate, Lipoide, Vitamine, Enzyme, anorganische Salze angegeben. Die stoffliche Zusammensetzung wird mit 45 % korpuskulären Stoffen wie Erythrozyten und 55 % Plasma angegeben. Der Wassergehalt der Blutkörperchen wird mit etwa 65 % und des Plasmas mit 90 % angegeben. Der Gesamtwassergehalt beträgt danach 78 %. Es wird beschrieben, dass in 0,01 g Blut 0,022 g Substanzen enthalten sind, die $CuCl_2$ bei der Kristallisation beeinflussen können. Für eine Blutkristallisation zu diagnostischen Zwecken werden auf der Glasplatte 0,01 g Blut; 1,5 g $CuCl_2$ in 6,0 cm³ bidestilliertem Wasser angesetzt, entsprechend dem Verhältnis von 1 : 150.

Die Beeinflussung des Kristallbildes durch Organextrakte erfordert nach diesen Untersuchungen ein anderes Verhältnis zwischen 1: 50 bis 1 : 200.

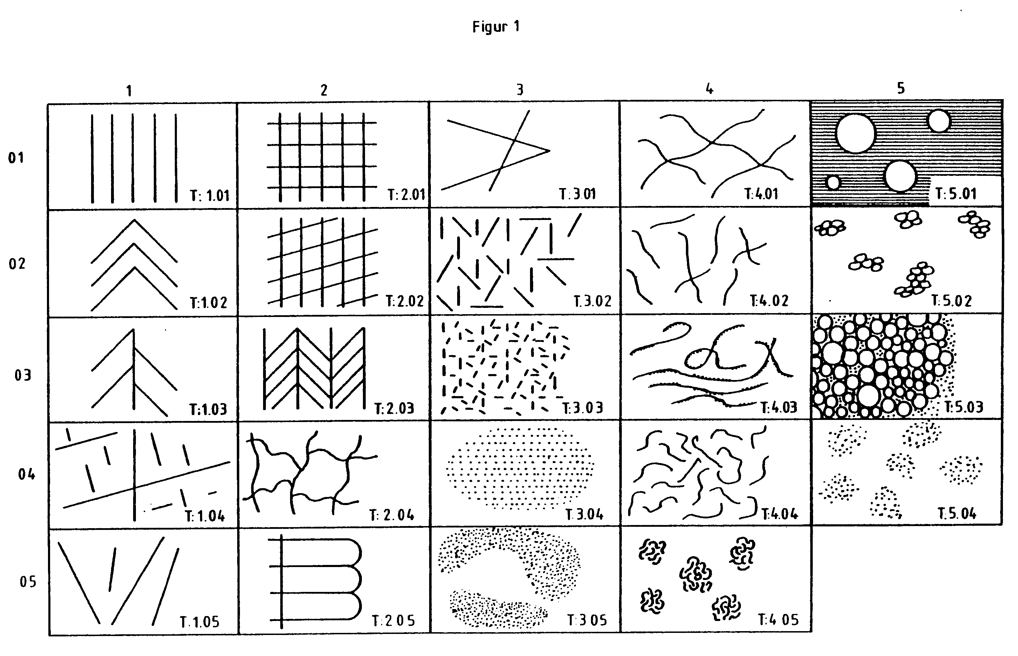

Die zahlreichen Freiheitsgrade bei der Ausbildung der $CuCl_2$- Blutkristallisation zeigt die Figur 1 der Autoren.

Es wird daraus auch die Streubreite dieser Methode bei der Anwendung auf dem medizinischen Sektor verständlich. Es werden Vergleiche der Texturen der Blutkristallisate gesunder und kranker Blutspender in Bezug auf die Ausbildung der Randzonen und die Veränderung der Texturen beschrieben, um zu einer diagnostischen Auswertung zu kommen. Diese wissenschaftliche Methode der Beeinflussung der Kristallisation von Salzen, insbesondere von $CuCl_2$ durch verschiedene Stoffe, insbesondere durch Blut, ist für eine sichere Diagnostik wegen der zu hohen Streubreite nicht geeignet. Dazu kommt die für die praktische Anwendung der Methode zu geringe Durchsatzkapazität wegen der zu langen Zeitdauer bis zum Vorliegen der Kristallisate. Eine Unsicherheit liegt auch in der Empfindlichkeit dieser Methode gegen äussere Einflüsse wie bei bereits geringen Schwankungen der physikalischen Bedingungen bei der Verdunstungszeit.

Der wesentliche Nachteil dieser Methode zur Diagnostik liegt auch darin, dass die Empfindlichkeit und damit die Aussagedeutlichkeit nicht ausreicht, weil der Zusatzstoff, wie das menschliche Blut, nur mittelbar die Kristallisation eines anderen Stoffes beeinflusst. Es können somit nur partielle Stoffe des Blutes, wie Aminosäuren, in Reaktion treten und die Kristallisation des anorganischen Salzes beeinflussen. Die Textur des gebildeten Kristallisates ist deshalb im wesentlichen auf die Lanzettenform mit negativem Feldbild beschränkt.

Diese Veröffentlichung beschränkt somit den Umfang und die Sicherheit der Diagnose, und diese hat sich somit auch nicht nennenswert in die Praxis eingeführt und ist daher als wissenschaftliche Methode zu bezeichnen mit nur geringer, gewerblicher Verwertbarkeit auch wegen des Zeitaufwandes. Ein Therapievorschlag ist aus diesem Stand des Fachwissens nicht hervorgangen, obwohl gerade auch dafür ein Bedürfnis der Medizin und der Öffentlichkeit besteht.

Die Wissenschaft und Technik hat seit fast 3 Jahrzehnten über die Erkenntnisse von Pfeiffer, "Empfindliche Kristallisationsvorgänge als Nachweis von Formkräften im Blut", Verlag E. Weise, 1935, verfügt, wonach Kupferchlorid in wässriger Lösung mit Zusätzen von pflanzlichen und tierischen Extrakten kristallisiert und ein zusatzspezifisches Gefügebild liefert, welches diagnostisch verwertbar ist.

Es bestand also seit dieser Zeit ein Bedürfnis der Fachwelt nach einer empfindlicheren, differentierbareren, leistungsfähigen Methode zur Lösung der Aufgabe, der Erzeugung von Kristallbildern für eine differentierte Frühdiagnose. Es hat offenbar ein Vorurteil der Praxis bestanden, die zu untersuchenden Substanzen direkt für die Erzeugung von Kristallbildern zu verwenden, weil morphologische Veränderungen der Ausganssubstanzen erwartet wurden, welche die Reproduzierbarkeit ausschliessen und zu Fehlerquellen und Streuungen in der Aussagefähigkeit führen. Ein solches Vorurteil ist durch das Verfahren der Erfindung überwunden, welches unter direkter Verwendung von anorganischen, organischen, biologischen, löslichen Stoffen aus einem Destillat und aus den damit zu einem Stoffgemisch vereinigten, löslichen Anteiles eines Kalzinates aus dem Rückstand der Destillation zur reproduzierbaren Kristallbildern im Kristallfeld mit typischen Kristalltexturen und Kristallformen führt.

In einer neueren Veröffentlichung, 5/84, Teleskop/HP-Heilkunde, beschreibt W. Prigge eine Methode zur vorklinischen Diagnose nach Professor Enderlein. Es wird Nativblut direkt mikroskopisch im Dunkelfeld betrachtet und abgebildet. Es wird der langsame Zerfall von bakteriellen und anderen Partikeln im Blutserum beschrieben und eine "unvorstellbare Vielzahl" von Formen festgestellt.

Diese Methode führt in eine andere Richtung, und diese hat das Verfahren der Erfindung nicht angeregt, sondern davon weggeführt.

Es ist die Aufgabe der Erfindung, flüssige Phasen zur Erzeugung von Kristallbildern aus diesen Ausgangsstoffen zu entwickeln, und diese Kristalltexturen und Kristallformen zum direkten Analogievergleich mit erzeugten Kristallbildern charakterisierbarer Stoffe zu verwenden.

Die Erfindung ist im Patentanspruch 1 festgehalten. Weitere Ausgestaltungen der Erfindung sind in den abhängigen Patentansprüchen enthalten.

Es ist von einer praktisch nicht vorhandenen Fehlerquote auszugehen. Diese wird dann noch weiter gesichert, wenn beispielsweise zwei oder noch mehr unabhängige Vergleichsanalogien durchgeführt werden und diese das gleiche Ergebnis in der Kennzeichnung oder Diagnose bestätigen.

Ein technischer Effekt des Verfahrens der Erfindung kann darin gesehen werden, dass das Kristallbild aus den Destillaten und den löslichen Anteilen des Kalzinates als Stoffmischung überraschend zu reproduzierbaren, typenbildenden Kristalltexturen und Kristallformen, insbesondere aus menschlichem Blut, Sekret, sonstigen Ausscheidungen die eine Frühdiagnose gestatten, oder aus anderen, organischen oder anorganischen Substanzen führt.

Ein anderer technischer Effekt des Verfahrens der Erfindung kann auch darin gesehen werden, dass die definierten, charakteristischen, ausgewählten Typen der Kristalltexturen und Kristallformen aufgrund der unerwarteten, ungleichmässigen Fliessgeschwindigkeit der Komponenten in der Lösung oder der Dispersion des Stoffgemisches aus dem Destillat und dem Kalzinat bei der Kristallisation aus dem als flächenhaft zu bezeichnenden Tropfen innerhalb des Kristallfeldes als reproduzierbare, örtliche definierte ringförmige Zonen der zugeordneten Organe und Funktionskreisen erzeugt werden.

Das Verfahren der Erfindung versteht unter den folgenden Begriffen die beschriebenen Definitionen. Unter Kristallfeld wird die auf den mikroskopischen Objektträger aus den Tropfen bei der Kristallisation erzeugte Kristallfläche verstanden. Unter Kristallbild wird die Gesamtheit der Kristalltexturen und der Kristallformen einschliesslich der Randzone nach der Kristallisation verstanden. Unter Kristalltexturen werden die bei der Kristallisation erzeugten, flächenhaften Aggregate der Kristallpartikel bzw. - Nadeln verstanden.

Freie Kristalltexturen sind solche, bei denen keine systematische Ordnung, wie bei einem Gitter, besteht, sondern die Lagerichtung einzelner Partikel zu einander unregelmässig ist.

Diese Partikel können sich kreuzen oder nicht berühren.

Lanzetten sind strichförmige Kristallisate, deren Längsausdehnung wesentlich grösser ist als ihre Seitenausdehnung. Die Kristallform entspricht dem Typ 0. 5. 4.

Unter "typische Figuren" der Kristalltexturen bzw. Kristallformen werden reproduzierbare, ausgewählte Darstellungen von Gebilden verstanden, die mikroskopisch und fotooptisch sichtbar sind, und die mathematisch oder beschreibend figürlich kennzeichenbar sind. Unter Reproduzierbarkeit der Kristalltexturen und Kristallformen wird die sichtbare, figürliche Formengleichheit oder Formenähnlichkeit bei mehrfacher, zeitlich unabhängiger Wiederholung der Erzeugung der Kristallisate aus der homogenen Mischung des Destillates und des löslichen Kalzinates aus mehreren Tropfen aus der gleichen oder vergleichbaren Ausgangssubstanz verstanden.

Das Verfahren der Erfindung kann in einer besonderen Ausführungsform ausgestaltet werden, wie das folgende Ausführungsbeispiel demonstriert.

Es kann die flüssige Phase der Ausgangsstoffe anstelle einer drucklosen Destillation einer Druckdestillation unterworfen werden.

Der Druck von 3,2 bar entspricht in einem speziellen Fall 145° C in dem Druckkessel. Dieser Druck bleibt bei der Destillation durch Zufuhr von Wärmeenergie aufrechterhalten. Die Druckentspannung erfolgt nach dem Ablassventil, wobei das Destillat in das Vorlagegefäss überläuft. Nach beendeter Destillation wird der Druck entspannt und der trockene Rückstand bei 140° C getrocknet und danach bei bis 950° C kalziniert und dann abgekühlt. Es wird danach das Kalzinat wie bei 140° C, dem Destillat hinzugegeben unter Angleichung der Temperatur durch Zischen. Es wird dann durch Schütteln oder Rühren das Stoffgemisch homogenisiert und zur Erzeugung des Kristallbildes verwendet.

Diese alternative Durchführung des Verfahrens der Erfindung mit Druckdestillation führt zur intensiven Emulgierung von in den Ausgangsstoffen enthaltenen Ölen und Fetten, wie bei Pflanzen mit hohem Anteil an ätherischen Ölen und Fetten, wie aus Laucharten oder stark eiweißhaltigen Organteile, wie Hirne. Diese alternative Ausgestaltung des Verfahrens der Erfindung führt durch die höhere Kalzinationstemperatur, wie beispielsweise bei 950°C, zu einer praktisch vollständigen Entfernung flüchtiger und vergasbarer organischer Komponenten und damit zu einer grösseren Porosität des Kalzinates, wie bei Pflanzen mit hohem Gehalt an Kieselsäure oder Erden. Diese alternative Durchführung des Verfahrens der Erfindung führt zur Adsorption von Ölen und Fetten aus der emulsionstrüben Lösung nach der Destillation.

Diese alternative Ausführungsform des Verfahrens der Erfindung ist vorzugsweise für die Herstellung von destillierten Urtinkturen aus Einzelpflanzen oder aus gemischten Pflanzen oder Pflanzenteilen, oder aus Organen, oder aus menschlichen oder tierischen Gewebeteilen zur Herstellung von Heilmitteln geeignet.

Das Verfahren der Erfindung wird durch das folgende Ausfürungsbeispiel erläutert. Es werden einer Person aus der Armvene 10 ml Blut entnommen, und diese Menge mit 10 ml tridestilliertem Wasser versetzt und zur intensiven Vermischung geschüttelt und in einen Glaskolben von 100 ml Inhalt eingeführt, der an einen Intensiv- Schlangenkühler angeschlossen ist mit nachfolgendem Vorlagegefäss.

Durch Erhitzen mittels Heizhaube oder Ölbad wird die Lösung zum Sieden gebracht, wobei die flüssige Phase in etwa 35 min überdestilliert.

Der in dem Glaskolben verbliebene Rückstand wird in einer Trockenkammer bei etwa 90°C in 60 min getrocknet bis zu einer spröden, dunklen Masse. Es wird danach diese Masse in einem Kalziniermuffelofen in einen Porzellantiegel von etwa 50 cm³ Inhalt eingeführt und bei bis auf etwa 750°C ansteigender Temperatur in einer Zeit von etwa 60 min kalziniert. Es wird danach die Zufuhr von Wärmeenergie abgestellt, das Kalzinat auf etwa 100°C in etwa 2 Stunden abgekühlt und mit dem Destillat in der Vorlage vermischt, danach intensiv durchgeschüttelt und nach einer Zeit von etwa 30 min zum Nachlösen löslicher Komponenten aus dem Kalzinat, vom ungelösten Resträckstand abfiltriert.

Die erzeugte Lösung wird auf einen mikroskopischen Glasträger mit 2 nebeneinander befindlichen Tropfen von je etwa 6 mm Durchmesser aufgebracht. Diese Tropfen werden bei Raumtemperatur von 20° bis 22°C auf dem Objektträger zur Trocknung gebracht, wobei sich vom Rand ausgehend bis zur Mitte ein flächendeckendes Kristallbild in Form von Kristalltexturen und Kristallformen ausbildet. Das Kristallfeld und die darin enthaltenen Kristalltexturen werden im mikroskopischen Dunkelfeld, oder bei schwachen Texturen im Phasenkontrast, oder im Differenzial- Interferenz-Kontrast nach Normanski, durch Analogievergleich mit in gleicher Arbeitsweise hergestellten Kristallisaten aus anderen Ausgangsstoffen verglichen und dadurch ausgewertet.

Das Verfahren der Erfindung hat eine bevorzugte Bedeutung zur Anwendung in der Frühdiagnose zum Erkennen von physiologischen und metabolischen Störungen, die zu manifesten Krankheitsbildern führen können, und die nach bekannten Standartmethoden erst in einem späteren Stadium der akuten Erkrankung erfassbar sind.

Das Verfahren der Erfindung hat auch eine Bedeutung für die qualitative virologische Diagnostik. Es kann eine Virusbelastung im menschlichen Körper im Kristallbild durch Punktanhäufung erkannt werden.

Das Verfahren der Erfindung bietet aber auch die Möglichkeit einer Verlaufskontrolle von diagnostizierten und therapierten Krankheitsbildern.

Die nach dem Verfahren der Erfindung aus dem Stoffgemisch des Destillates und des löslichen Teiles des Kalzinates erzeugten Kristallisate gestatten in ihrem Inhalt eine diagnostische, oder strukturelle, oder therapeutische Zuordnung zu anorganischen, oder zu organischen oder zu physiologischen Phänomenen, d.h. auch zu bestimmbaren Krankheitsbildern und zu diesen entsprechenden Heilsubstanzen in nach dem Verfahren der Erfindung hergestellten Heilmitteln.

Das folgende Ausführungsbeispiel demonstriert die erfindungsgemässen Parameter zur Herstellung des Heilmittels nach dem Verfahren der Erfindung.

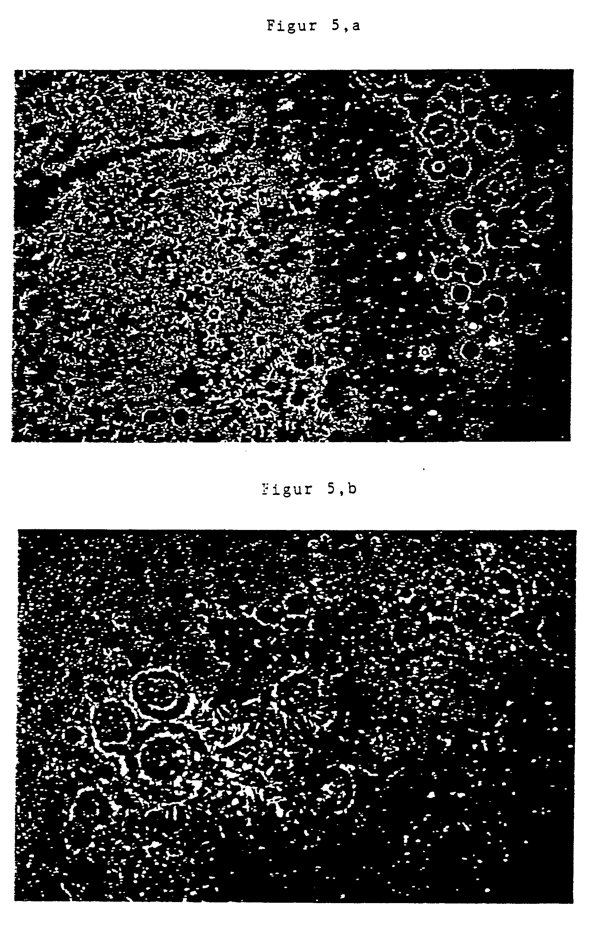

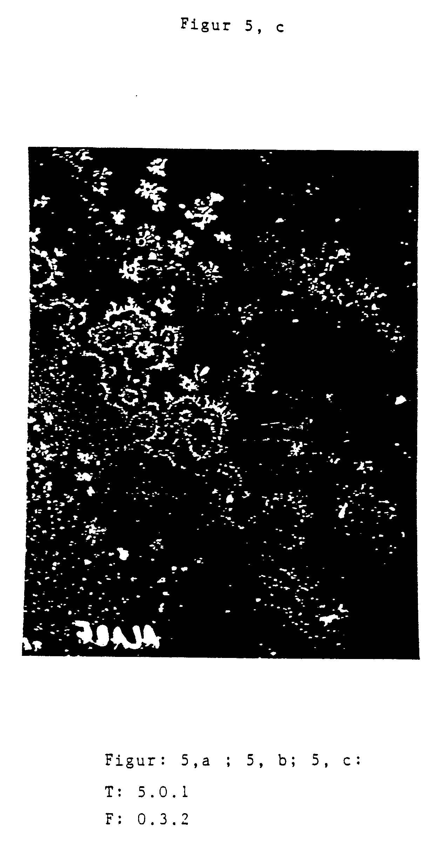

Durch Analogievergleich ist nach dem Verfahren der Erfindung als aktuelles Krankheitsbild Leukämie diagnostiziert. Das dieser Krankheit zugeordnete Kristallbild ist mit Kristalltexturen und Kristallformen in Figur 5 dargestellt.

Zur Herstellung des Heilmittels wird durch Analogievergleich des Kristallbildes dieser Krankheit mit den Kristallbildern von Pflanzen oder anderen Stoffen die formale Übereinstimmung der Kristalltexturen und Kristallformen oder die höchstmögliche Ähnlichkeit festgestellt.

Aus den durch diesen Analogievergleich ermittelten Pflanzen oder Stoffen wird durch Vergärung und durch Destillation und Kalzinierung des Rückstandes nach dem Verfahren der Erfindung und nach der Herstellung der homogenen Stoffmischung nach erfolgter Abfiltrierung von unlöslichen Restsubstanzen die Urtinktur hergestellt. Sofern das Kristallbild Kristalltexturen und Kristallformen mehrerer Krankheitsbilder offenbart, wird der Analogievergleich auch für die weiteren Kristalltexturen und Kristallformen in gleicher Weise durchgeführt zur Bestimmung der diesen weiteren Krankheitsbildern zugeordneten Urtinkturen. Es werden dann aus den entsprechenden Pflanzen oder Pflanzenteilen die Urtinkturen hergestellt und unvermischt, oder zu Komplexen vermischt zur Therapie verwendet. Es ist dabei notwendig, nur solche Urtinkturen miteinander zu einem Komplex zu mischen, deren Kristalltexturen und Kristallformen ähnlich sind.

Das folgende Ausführungsbeispiel demonstriert die Herstellung eines Kristallbildes mit Kristalltexturen und Kristallformen aus einer nach dem Stand der Technik als in Wasser unlösliche und daher darin nicht kristallisierende Substanz.

Solche Stoffe sind insbesondere anorganische Metalle und organische Öle die keine Ionen abgeben sollen. Es wird beispielsweise Blattgold als bekannte, wasserunlösliche Substanz in tridestilliertem Wasser am Rückflusskühler in Abhängigkeit von der Zeit mehrere Tage destilliert und nach Ablauf des Zeitabschnittes in die Vorlage überdestilliert.

Zu diesem beispielsweisen Versuch werden auch Vergleiche zu heptadestilliertem Wasser angestellt zur Demonstration der Reproduzierbarkeit der Ergebnisse, und es werden Blindversuche mit diesem Wasser angestellt.

Das Destillat wird ohne ein Kalzinat auf den mikroskopischen Objektträger aufgebracht, und die Bildung eines Kristallisates nach dem Verfahren der Erfindung geprüft.

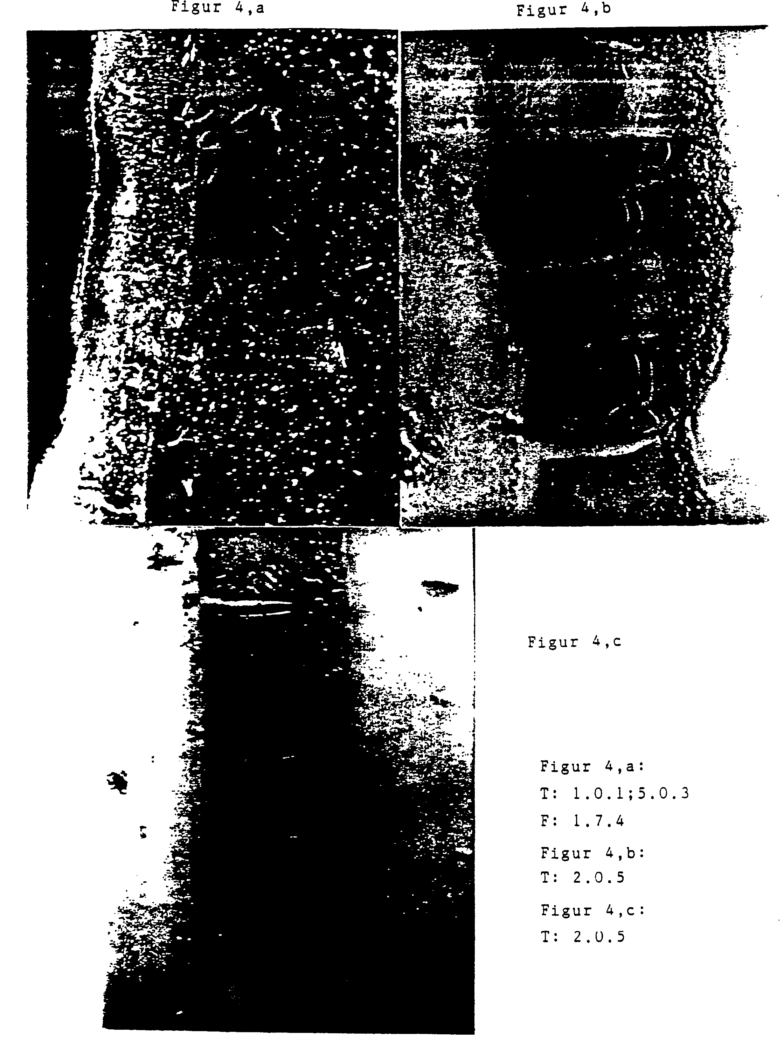

Es wird überraschend die Bildung eines reproduzierbaren Kristallbildes festgestellt, wie in Figur 4 dargestellt.

Es zeigt sich beispielsweise für Gold in Form von Blattgold nach dem 1. Tag eine Bandstruktur am Tropfenrand mit vagabundierenden, keilförmigen, freien bzw. sich kreuzenden Lanzetten. Diese entsprechen Figur 4, a.

Es bildet sich nach dem 5. Tag eine bandförmige Ausprägung in Form der Oberfläche eines ausgetrockneten Lehmbodens. Siehe Figur 4, b.

Es bildet sich nach dem 7. Tag die vereinfachte Form des 5. Tages in netzförmiger bzw. leiterförmiger Kristalltextur. Siehe Figur 4 c. Dieses Beispiel demonstriert, dass nach dem Verfahren der Erfindung auch der diagnostische Nachweis von Feinstspuren von an sich wasserunlöslichen Stoffen möglich ist.

Es ist dazu erforderlich, dass aus definierten Substanzen die Kristallbilder in Form der Kristalltexturen und Kristallformen nach dem Verfahren der Erfindung jedoch ohne ein Kalzinat zur Vergleichsanalogie zur Verfügung stehen.

Es ist nach dem Verfahren der Erfindung also möglich, in Substanzen Spuren von Stoffen, wie Schwermetallen, durch Analogievergleich qualitativ festzustellen. Dieses überraschende Phänomen beruht offenbar darauf, dass mikrofeine Spuren von an sich wasserunlöslichen Stoffen in Lösung gehen, wenn diese in Abhängigkeit von der Zeit mit der flüssigen Phase, wie Wasser, in Verbindung stehen. Es ist überraschend, dass diese Stoffe, wie Schwermetalle, beim Inlösunggehen in der wässrigen Phase Ionen bilden, denn nur diese können aus dem Destillat Kristalltexturen und/oder Kristallformen bei der Herstellung des Kristallisates bilden.

Es werden in diesem, nach dem Verfahren der Erfindung erzeugten Blutkristall die Texturen und Formen aller Organe optisch sichtbar, die pathogen oder funktionell sind. Das Verfahren der Erfindung offenbart Parameter der Fläche (1) der Form (2) der Position (3).

Der Flächen-Parameter (1) stellt die texturelle Identifikation physiologischer Störungen von Organen und physiologischen Funktionskreisen (z. B. Verdauungstrakt) an beliebiger Position im Kristallfeld dar. Der Form-Parameter (2) stellt die Identifikation erkrankter Organe anhand der Kristallisatform in beliebiger Position im Kristallfeld dar. Der Positions-Parameter (3) stellt die organotopische Zuordnung des nach dem biogenetischen Grundsatz des Keimblattes geordneten ekto, meso-, entodermalen Bereiches dar, wobei sich beispielsweise im mesodermalen Bereich vom Rachenraum bis zum Mastdarm vom Rand des Kristallfeldes nach innen bis gegebenenfalls zum zentralen Mittelpunkt die Kristalltexturen und Kristallformen ausbilden.

Die Abbildungen zeigen in
Figur 1: Kristalltexturen,
Figur 2: Kristallformen,
Figur 3: Topologie-Modell des menschlichen Organfeldes.

Das im Kristallfeld nicht abgebildete Organ ist als pathologisch unauffällig zu bezeichnen.

Das positiv abgebildete Organ ist pathologisch-auffällig im Sinne einer Hyper- oder Hypoergie, wie eine Infektion, entzündliche Degeneration, Geschwür- und Geschwulstbildungen, zu bezeichnen.

Das in einem positiven Umfeld negativ durch Aussparung abgebildete Organ ist degenerativ im Sinne eines progressiven Funktions- und Vitalitätsverlustes. Seine physiologische Situation ist schwer beeinflussbar.

Das Verfahren der Erfindung gestattet, diese ausgewählten Typen mikrooptisch und fotografisch sichtbar zu machen. Die objektive Zuordnung zu definierten Organen ist durch den Analogievergleich von Kristalltexturen und Kristallformen des zu untersuchenden Blutes zu denen von definierten Organfeldern und physiologischen Störungen (Krankheiten), sowie Heilmitteln ermöglicht.

Figurenerläuterung.
Figur 1: Kristalltexturen,

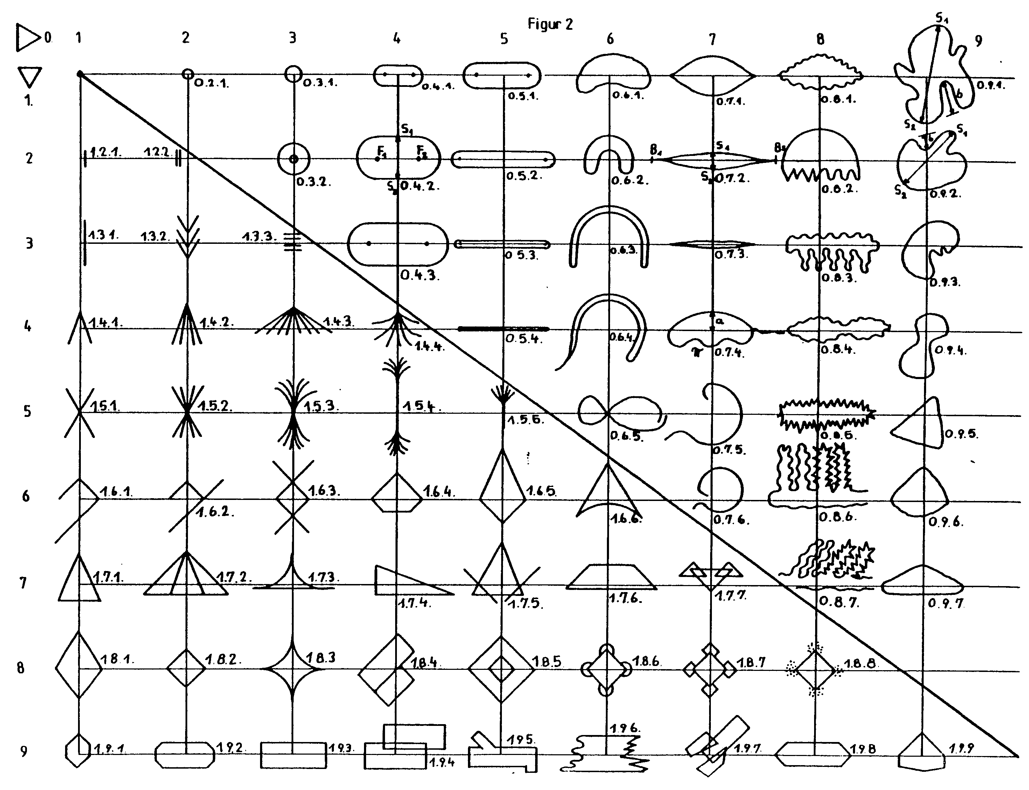

Figur 2: Kristallformen,

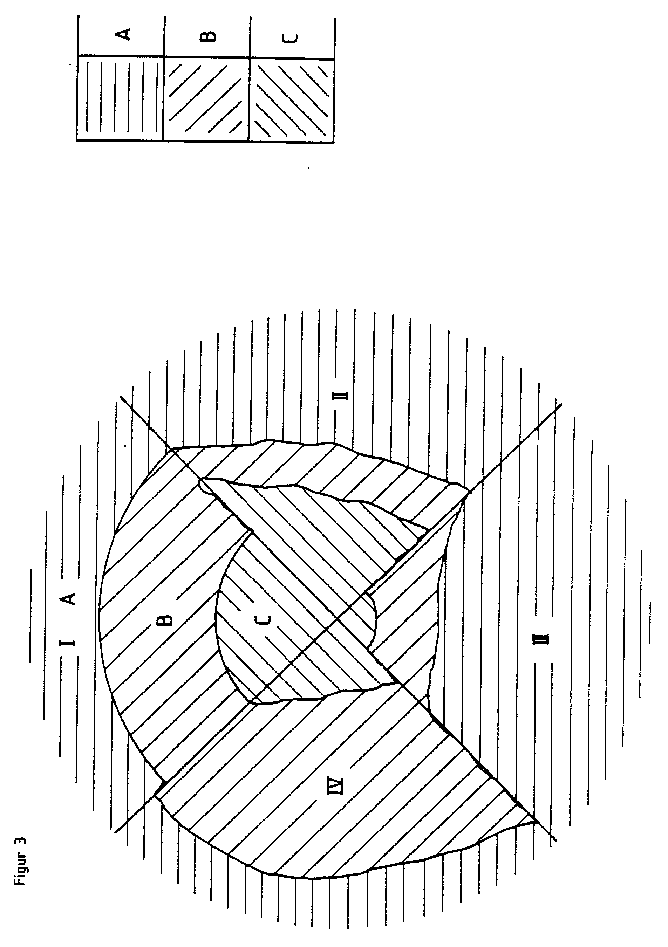

Figur 3 zeigt die Topologie der menschlichen Keimblätter A, B, C.

Es bedeuten A = entoderm; B = mesoderm; C = ektoderm..

Bei gesundem Blut ist im Kristallbild die Verteilung der Seiten A : B : C = 1 : 1 : 1. Je nach pathologischer Belastung der einzelnen Keimblätter kann A, B, C allein oder mit einem anderen Keimblatt den gesamten Raum zu ungunsten der anderen Keimblätter einnehmen.

Normale Typen für Kristallformen:

(A)  Kristalltextur: 1.0.1; 1.0.2; 3.0.4; 5.0.1
     Kristallformen: 0.3; 0.4; 0.5; 0.7,
(B)  Kristalltextur: 4.0.1,
     Kristallformen: 1.7; 1.8, 1.9; 0.8; 0.9,
(C)  Kristalltextur: 1.0.2; 1.0.3; 2.0.3; 5.0.3,
     Kristallformen: 1.3; 1.5.

In dem Sektor 1 ist die normale Verteilung der Keimblätter eines gesunden Blutes: A = D = C, bezogen auf den Radius, dargestellt.
In dem Sektor II ist die Verteilung der Keimblätter bei Erkrankung des Nerversystems dargestellt: A > B < C.
In dem Sektor III ist die Verteilung der Keimblätter bei Erkrankung des Verdauungssystems von Leber und Pankreas dargestellt: A > B > C.
In dem Sektor IV ist die Verteilung bei rheumatischen und chronisch- entzündlichen Erkrankungen dargestellt:
A < B > C.
Die dargestellten Sektoren I bis IV stellen eine Auswahl typischer Keimblattverteilung bei entsprechenden Krankheitsbildern dar.

Figur 4:   Textur von Blattgold (Au),
4, a:      Rückdestillation nach 1 Tag,
4, b:      Rückdestillation nach 5 Tagen,
4, c:      Rückdestillation nach 7 Tagen,
zu 4, a:   Ausbildung des Bandes, T : 1.0.1 ;
           5.03.F : 1.7.4,
zu 4, b:   Ausbildung des Bandes mit Fissuren,
           T : 2.0.5;
zu 4, c:   Vereinfachung der Ausbildung von 4,
           b, leiterförmig, T : 2.0.5,

Die Figuren 4 demonstrieren den Nachweis, dass chemisch, analytisch, nicht nachweisbare Metallspuren im Kristallbild sichtbar und zu kennzeichnen sind.

Figur 5, a:   Kristallbild: Texturen des Blutes
              eines Leukämie-Kranken,
              Formentyp: 0.3.2.
              Texturtyp: 5.0.1,
Figur 5, b:   wie Figur 5, a, jedoch das Blut eines
              anderen Patienten.
Dieser Vergleich der Figuren 5, a und 5, b bringt den Nachweis, dass bei der gleichen Erkrankung die gleichen, charakteristischen Kristalltexturen und Kristallformen reproduzierbar ausgebildet werden.

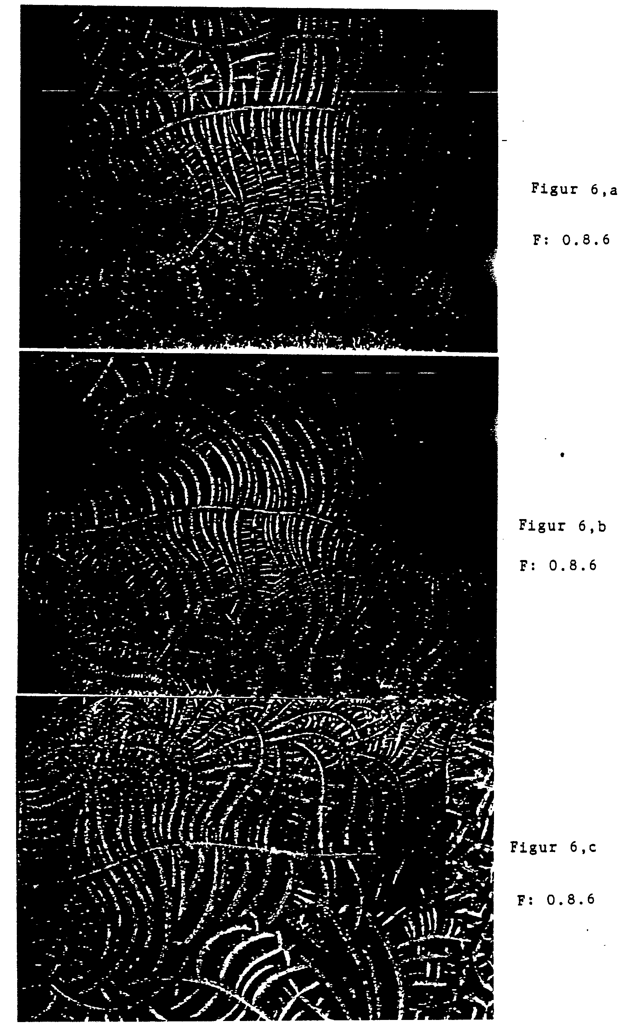

Figur 6, a, b, c: Kristalltexturen und
              Kristallformen des Blutes eines
              Patienten.

Diese Kristallbilder demonstrieren die Reproduzierbarkeit bei der Kristallisation aus der gleichen Dispersion nach dem Verfahren der Erfindung (gleiche Zeit, gleiche Temperatur).
Formentyp: 0.8.6.

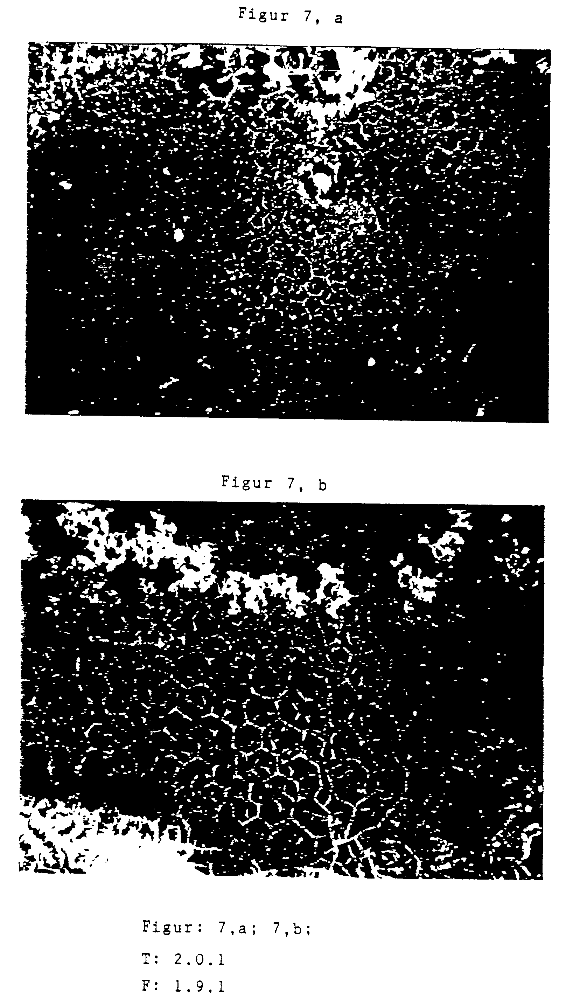

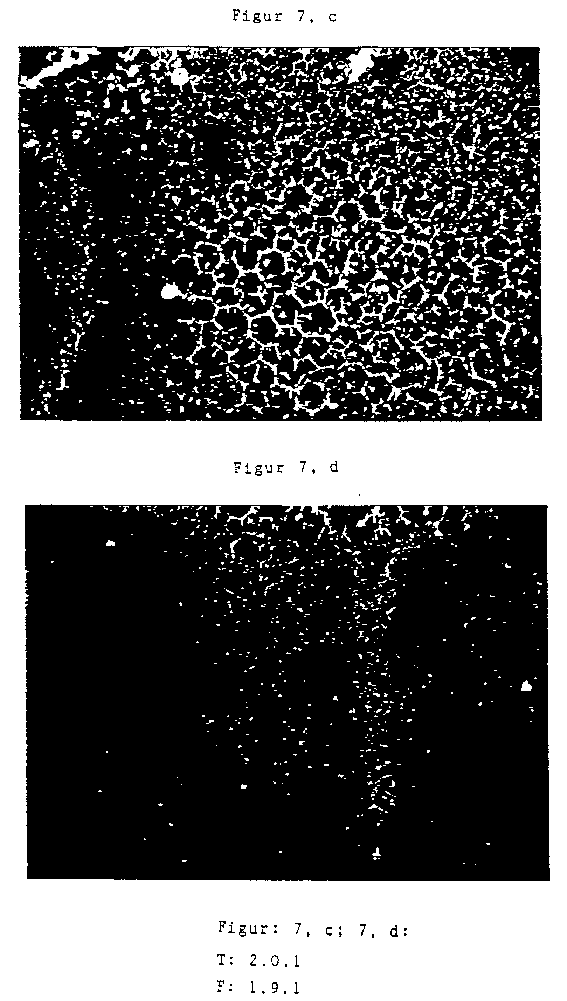

Figur 7, a, b, c, d: Kristalltexturen und
              Kristallformen aus der Pflanze Amygdalae amarae (Bittermandel) nach dem Verfahren der Erfindung.

Diese Kristallbilder demonstrieren ebenfalls die Reproduzierbarkeit aus der gleichen Lösung nach dem Verfahren der Erfindung.
Texturtyp: 2.0.1. Formtyp: 1.9.1.

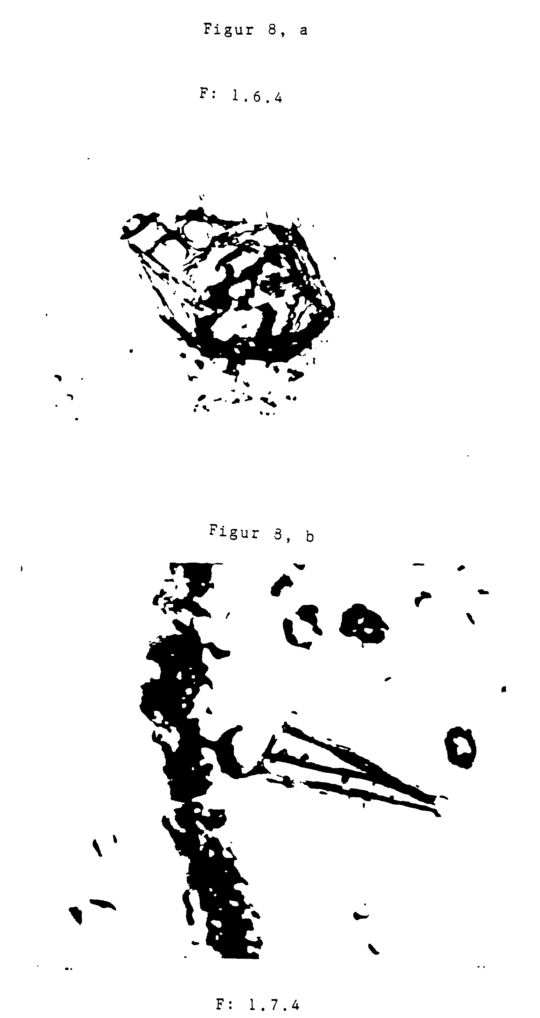

Figur 8, a:   Nachweis einer Kristallform des
              menschlichen Herzens, Formtyp:
              1.6.4
Figur 8, b:   Nachweis der Kristallform der
              menschlichen Niere: Formtyp: 1.7.4.

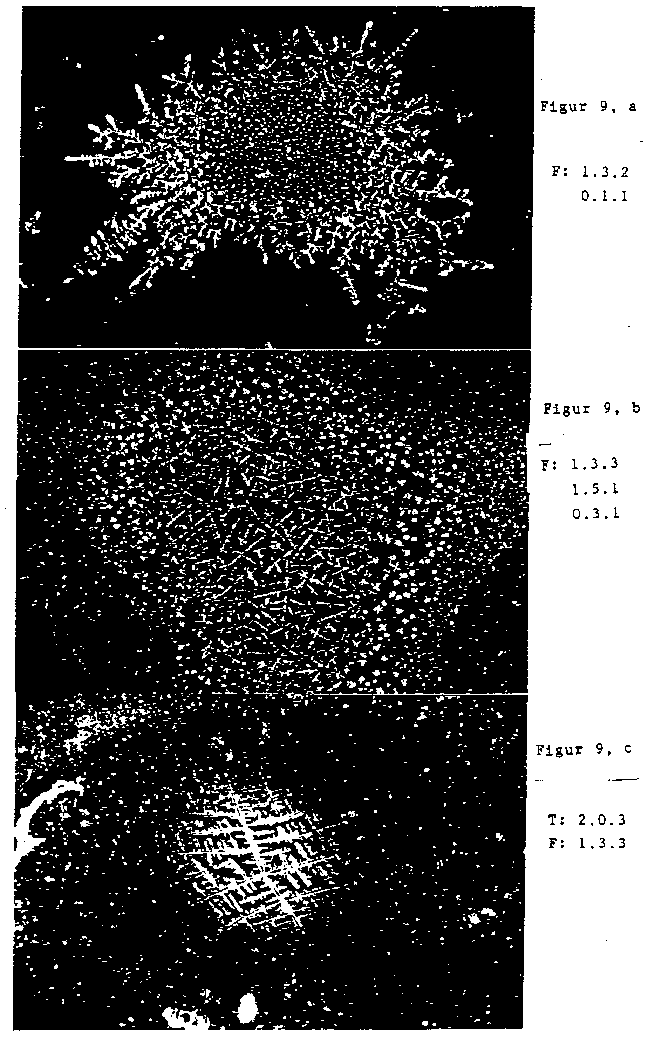

Figuren 9, a, b, c: Quellwasser, Provenienz: Natürliches, im Brunnen gefasstes und freifliessendes Mineralwasser, Quelle Römäus, Villingen.
Das Verfahren der Erfindung gestattet also einen Nachweis physikalischer Effekte auf Substanzen, wie beispielsweise Wasser, durch die optische Sichtbarmachung. Das Verfahren der Erfindung stellt also auch ein analytisches Verfahrem zur Kennzeichnung physikalischer Effekte dar mit hoher Analysenempfindlichkeit.
Die Sichtbarmachung dieses physikalischen Effektes erfolgt durch Analogievergleich zu dem gleichen Wasser vor der Durchführung der physikalischen Behandlung. Der Vergleich erfolgt also durch die optische Betrachtung der "Blindprobe".
Es ist offenbar eine Ausrichtung der Struktur des Wassers im molekularen Bereich erfolgt und durch das Verfahren der Erfindung sichtbar gemacht. Wesentlich ist dabei, dass diese, durch die Einwirkung physikalischer Kräfte "gerichteten" Strukturen, des Wassers als Destillat übergehen, und diese somit als "Quasi-Elektrolyte" zu bezeichnen sind. Diese geordneten Strukturen werden nach dem Verfahren der Erfindung als Kristallbilder in Form von Kristalltexturen und Kristallformen optisch sichtbar gemacht. Es werden die folgenden, ausgewählten Typen von Kristalltexturen und Kristallformen festgestellt:
     Figur 9, a.
     Form: 0.1.1. 1.3.2.
     Kristallisat nach 7 Tagen.

     Figur 9, b:
     Form: 1.3.3. 1.5.1. 0.3.1.
     Kristallisat nach 14 Tagen.

     Figur 9, c:
     Form: 1.3.3. 2.0.3.
     Kristallisat nach 21 Tagen.

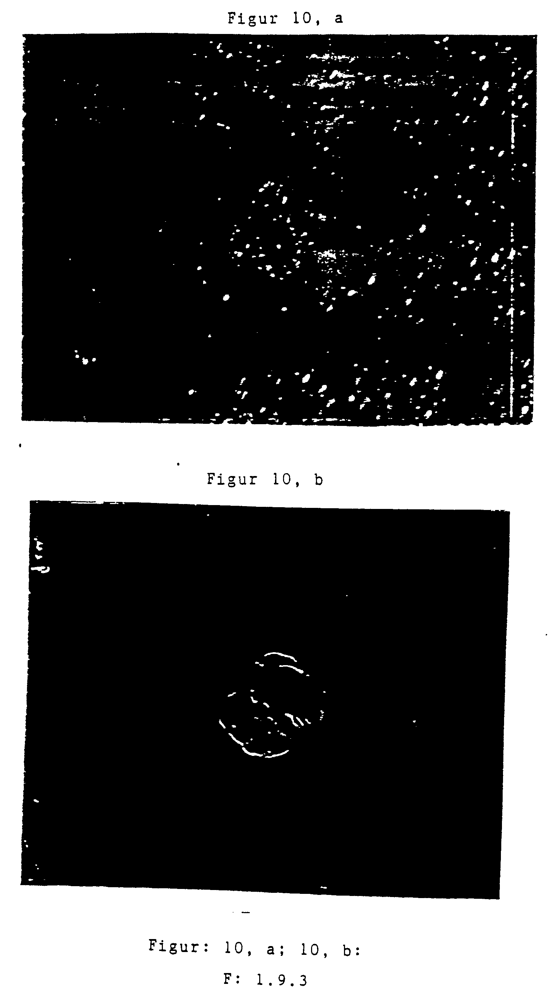

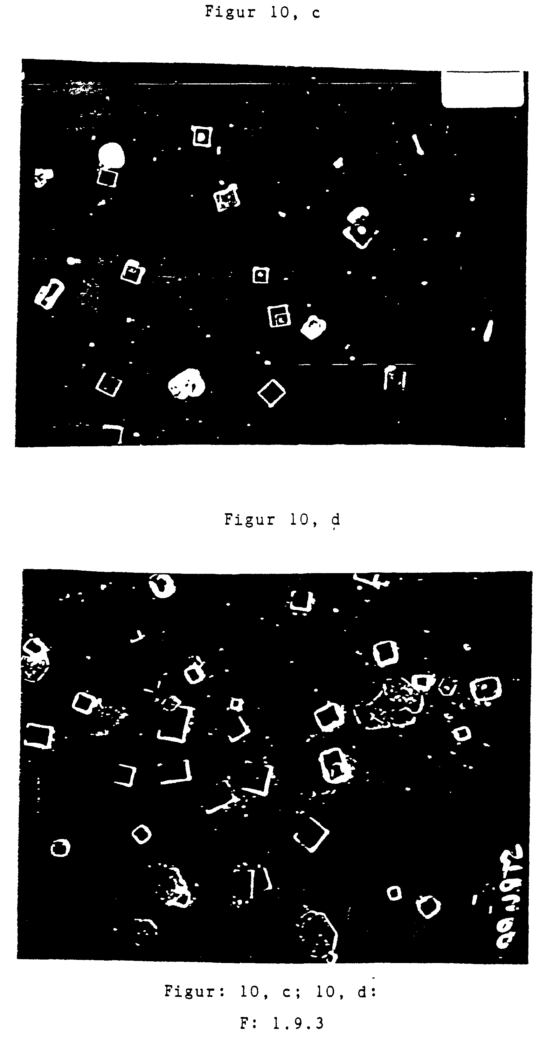

     Figur 10, a, b,c , d: Kristallbild der
              Krankheit Morbus Bechterew
              (Wirbelgliederentzündung).

Diese Figuren demonstrieren den Krankheitsverlauf innerhalb dieser Zeitspanne mit zunehmender Versteifung der Wirbelsäule. Diese Kristallbilder werden zum Nachweis der Reproduzierbarkeit bei definierten Krankheiten bei verschiedenen Patienten und bei unterschiedlichen Entwicklungsstadien in den folgenden Figuren dargestellt.

Figur 10, a: männlich, 27 Jahre, entzündliches Stadium, Lähmungsgefühle,

Figur 10, b: männlich, 35 Jahre, Versteifung der Halswirbelsäule,

Figur 10, c: männlich, 42 Jahre, Versteifung der gesamten Wirbelsäule mit starker Einschränkung der Kopfbewegung.

Figur 10, d: männlich, 67 Jahre, vollständige Versteifung der Wirbelsäule, Bewegungsunfähigkeit des Kopfes, alle Kristallformen: F 1.9.3.

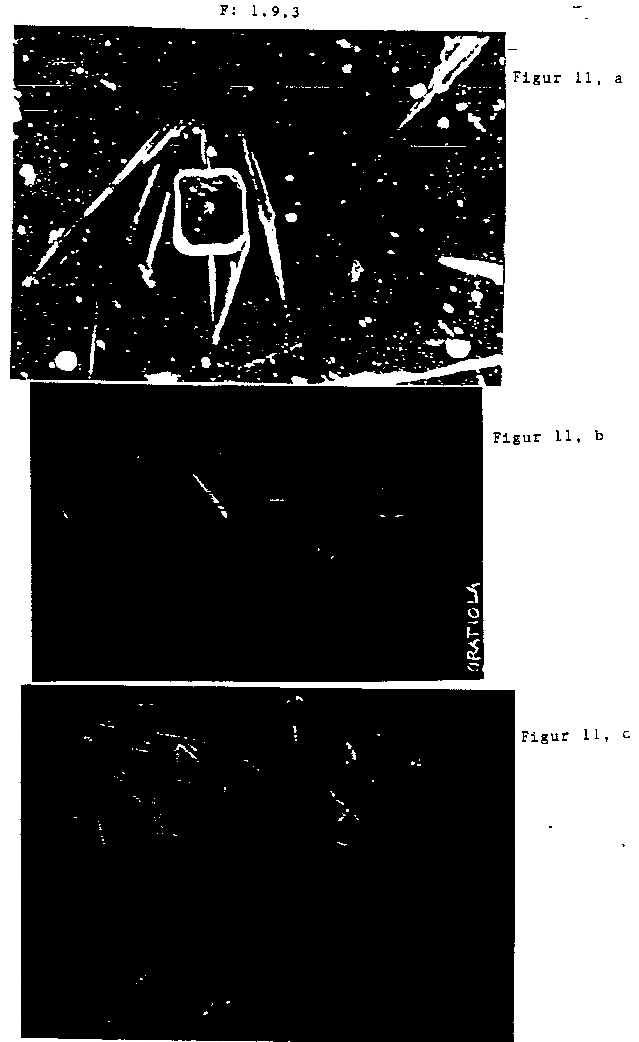

Figur 11: Heilmittel nach dem Verfahren der Erfindung zur Therapie der Krankheit nach den Figuren 9, a - d. Der Analogievergleich der Kristalltexturen und Kristallformen erfolgt zur Festlegung der auszuwählenden Pflanzendestillate oder der auszuwählenden Pflanzen für die Herstellung eines Heilmittels nach anderen Verfahren.

Figur 11, a: Arnica montana (Bergwohlverleih), Form: 1.9.3.

Figur 11, b: Gratiola officinalis (Gottesgnadenkraut), Form: 1.9.3,

Figur 11 c: Aconitum mapellus (Echter Strumhut). Form: 1.9.3.

Diese photografischen Abbildungen zeigen also die Sichtbarmachung der Kristallisate nach dem Verfahren der Erfindung in Form der reproduzierbaren, charakteristischen, ausgewählten Typen der Kristalltexturen und Kristallformen zur Anwendung bei der Frühdiagnose von Krankheitsbildern, bei der Kennzeichnung von physikalischen Vorgängen und bei der kristalloptischen Bestimmung von Stoffen und Stoffspuren. Für eine Übersichtsvergrösserung zur Identifizierung der Kristalltexturen und grösserer Kristallformen wird insbesondere eine 25 bis 60- fache Vergrösserung verwendet.

Zur Detaillierung kleinerer Kristalltexturen sowie von punktförmigen Kristallformen wird vorzugsweise eine 160 bis 450- fache Vergrösserung verwendet.

Die Bildung der Kristallisate wird beispielsweise wie folgt beschrieben:

Zeit: t = 0 sec: Auftropfen einer aus dem Destillat und dem Kristallisat gebildeten, homogenen Lösung auf einen mikroskopischen Objektträger bei einer Raumtemperatur von 20° C und einer relativen Luftfeuchtigkeit von 57 %.

Zeit: t = 20 sec: Bildung von punktförmigen Kleinformen von 0,0153 bis 0,022 mm Durchmesser mit einer Dichte von 4 Punkten je mm².

Zeit: t = 5 min: Bildung von Punktfeldern mit 0,04 mm Durchmesser mit einer Dichte von etwa 22 Punkten je mm².

Zeit. t = 15 min: Bildung von Flächenkreisen bis zu 0,08 mm Durchmesser mit einer Dichte von 2 bis 4 Punkten je mm².

Die Messung erfolgte am linearvergrössernden Fernsehmonitor durch Verhältnisbildung zwischen Kristallfeld auf dem Objektträger und linearvergrösserten Fernsehbild desselben: Masstab: 1 : 140.

Zeit: t = 40 min: Plötzlicher Beginn der fast gleichzeitigen Kristallisation des gesamten Tropfenfeldes, die innerhalb von 90 bis 120 sec abgeschlossen ist.

Die Wachstumsgeschwindigkeit der einzelnen Kristallformen, oder Lanzetten, oder Nadeln beträgt 0,08 mm / sec mit einer Streuung von ± 25 %.

Zeit t = 43 min: Die Kristallisation und damit die Ausbildung des Kristallbildes ist vollständig abgeschlossen, und es erfolgt eine Aushärtung des Kristallisates. Die beispielsweise beschriebenen Zeitangaben können in der Praxis um ± 10 % abweichen. Das Kristallfeld, das aus der Auftropfung der homogenen Lösung aus dem Destillat und dem löslichen Teil des Kristallisates gebildet ist, hat einen Durchmesser von 6 mm.

Die einzelnen Segmente des Kristallfeldes nehmen in der Regel etwa den folgenden Radius, vom Umkreis bis zur Mitte gerechnet, ein:

Entoderm: 1 - 2 mm; Mesoderm: 1 - 3 mm; Ectoderm: 0 - 3 mm.

Die Grenzwerte der bevorzugten Luftfeuchtigkeit betragen etwa 47 bis 60 %.

Sofern die Temperatur im Einzelfall geringer ist als 20° C, z. B. 17 - 18° C, erhöht sich die Dauer Kristallisation um etwa 10 Minuten.

Sofern im Einzelfall die Temperatur über 20° C z. B. bei 24° C, liegt, verkürzt sich die Dauer der Kristallisation auf bis etwa 30 min.

Die Wachstumsgeschwindigkeit beträgt im Augenblick der effektiven Kristallisation beim Zeitpunkt: t = 40 min, gerechnet vom Zeitpunkt: t = 0 min, im Augenblick des Auftropfens auf den mikroskopischen Objektträger, 0,08 mm /sec als Durchschnittswert. Es können einzelne Lanzetten Wachstumsgeschwindigkeiten von 0,5 mm /sec für die Dauer bis zu 3 sec erreichen. Es können andere Lanzetten nur 0,01 mm/sec über einen Zeitraum von 8 - 15 sec wachsen.

Unter der Wachstumsgeschwindigkeit wird die Fortpflanzungsgeschwindigkeit der Kristalltextur oder der Kristallform im Kristallisationsfeld verstanden. Es handelt sich also nicht um die Fortbewegung einzelner Massepartikel, sondern um die Veränderung der Dimension des Kristallbildes in der effektiven Länge in mm in Abhängigkeit von der Zeit in Sekunden.

Das Verfahren der Erfindung verwendet das Phänomen als technischen Effekt, dass die Kristalle mit ihrer jeweiligen Organinformation in der homogenen Lösung oder Dispersion während der Kristallisation an einem definierten, reproduzierbaren "Ort" als geometrisch bestimmbare Fläche kristallisieren.

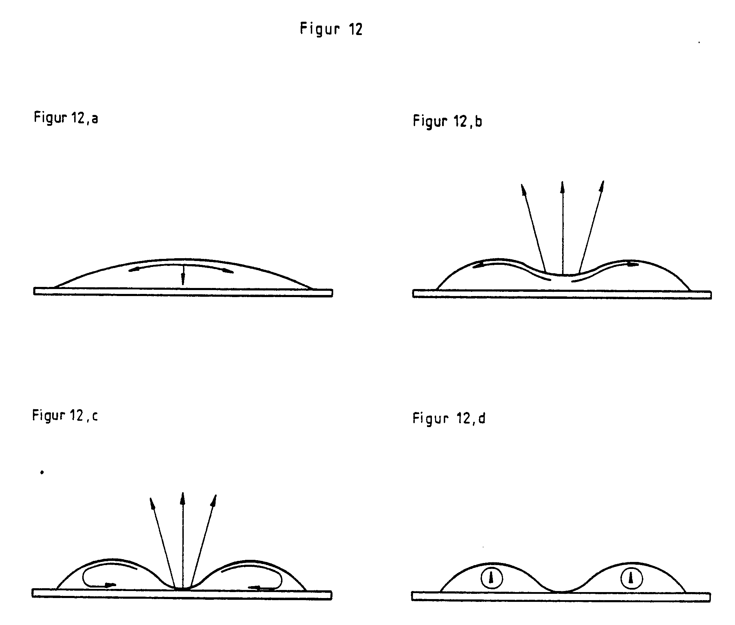

Für diesen Effekt, dass bestimmte Kristallformen an bestimmten "Orten" des Kristallfeldes optisch zu ermitteln sind, mag es verschiedene, theoretische Erklärungen geben. Eine physikalische Erklärung ist die folgende: Siehe Figur 12.

Bei der Auftropfung der Lösung oder Dispersion bildet sich eine Tropfenform auf dem mikroskopischen Objektträger. In der Tropfenmitte hat dieser Tropfen seine maximale Dicke (höchste Erhebung) und dadurch seine grösste Zugspannung in der gegenüber der Umluft gebildeten Aussenhaut. Im Scheitelpunkt sind die Zugkräfte am grössten, und die Adhäsionskräfte am geringsten. Es kommt dadurch an dieser Stelle zur schnelleren Verdampfung der wässrigen Phase. Die sich in Lösung befindlichen Stoffpartikel wandern dadurch in die Randzonen, in welchen sich die flüssige Phase länger erhält. Durch die schnellere Abdampfung bildet der Scheitelpunkt in seiner konvexen Aufwölbung eine konkave Einbuchtung aus. Das Volumen der flüssigen Phase unterhalb dieser Einbuchtung verringert sich. Es werden dadurch die gelösten Partikel angeregt, sich in die Richtung der noch flüssigen Randzonen zu bewegen. Es kommt deshalb in diesen Randzonen zu einer Vermehrung an kristallisierenden Partikeln, wobei das Tropfenzentrum im allgemeinen eine verminderte Kristalldichte aufweist.

Es ist durchaus möglich, die "Zugspannung" als Oberflächen- oder als Grenzflächenspannung zu messen.

Da die Organformen in einzelnen Kristallformen kristallisieren, die gegenüber den Kristalltexturen eine höhere Dichte und Masse aufweisen, sind diese unter 1 % in der Mittelzone des Kristallfeldes optisch festzustellen. Diese Kristallformen befinden sich in der flüssigeren Mittelzone (Zwischenzone), in welcher diese noch ausreichende Zeit zum massenreicheren Auskristallisieren finden, da die Abdampfung langsamer erfolgt. Diese grösseren Kristalle sind auch nicht in den Randzonen optisch festzustellen. Dies liegt daran, dass in diesen Randzonen die noch langsamere Abdampfung zu einer hohen Partikeldichte führt, sodass diese meistens in dicken, geschlossenen Flächenrastern auskristallisieren und nur in seltenen Fällen Raum für eine Kristallisation von Einzelformen frei lassen. Die räumliche Ausbreitung der Stoffpartikel bei der Kristallisation ist in der Figur 12 a,b,c,d dargestellt.

Es zeigt die Figur 12,a die Darstellung der Richtung der Zugspannung in der flüssigen Phase des Tropfens.

Es zeigt die Figur 12, b das Maximum der Abdampfung der flüssigen Phase aus dem Tropfen.

Es zeigt die Figur 12, c die Fliessrichtung der Stoffpartikel in der flüssigen Phase mit konvexer Einbuchtung.

Es zeigt die Figur 12, d das Maximum der Anhäufung der Stoffpartikel in der Mittelzone des Kristallisationsfeldes. Diese theoretische Betrachtung der "Wanderung" der Stoffpartikel in der flüssigen Phase des Tropfens bei der Abdampfung bis zur Kristallisation an einem definierten, reproduzierbaren "Ort" auf der Fläche des Kristallfeldes ergibt sich aus der experimentellen Beobachtung. Diese Betrachtung schliesst andere theoretische Erörterungen wie eine energetische Berechnung aus der Reaktionskinetik nicht aus.

**Figurenerläuterung.**

| | |
|---|---|
| Fig. 1 | Kristallfelder: Texturen |
| Fig. 2 | Kristallfelder: Formen |
| Fig. 3 | Modell der Topologie der menschlichen Keimblätter |
| Fig. 4a | Textur des Goldkristallisats: nach einem Tag der Rückdestillation |
| Fig. 4b | nach dem 5. Tag der Rückdestillation |
| Fig. 4c | nach dem 7. Tag der Rückdestillation |
| Fig. 5a | Kristallformen und -texturen aus dem Blut eines Leukämikers. (Frau, 36 Jahre). |
| Fig. 5b | Kristallformen und -texturen aus dem Blut eines Leukämikers. (Mann, 19 Jahre). |
| Fig 5c | Kristallformen und -texturen aus dem Blut eines an Leukämie verstorbenen Mannes (34 Jahre), etwa zehn Tage vor seinem Tode. Alle Kristallfelder haben folgende Typisierung: F: 0.3.2./ T: 5.01 |
| Fig. 6 a- c | Vier einzelne Kristallisationen aus demselben Destillat-Calcinat-Gemisch des Bluts desselben Menschen; entnommen in vier verschiedenen Proben; kristallisiert zur selben Zeit unter denselben Temperaturbedingungen. |
| Fig. 7 a - d | Vier Einzelkristallisationen aus dem homogenen a - d Destaillat-Calcinat-Gemisch der Pflanze Amygdala amara, Bittermandel. Typ: T : 2.01, F : 1.9.1 |
| Fig. 8a | Kristallform: Organ Herz, Typ: F: 1.7.4 |
| Fig. 8b | Kristallform: Organ Niere. Typ: F: 1.6.4 |
| Fig. 9a | Romäus-Quelle, magnetisches Gleichfeld 7 Tage, Typ: T: 3.02 |
| Fig. 9b | 14 Tage, Typ: F: 1.3.3 |
| Fig. 9 c | 21 Tage, Typ: F:0.9.6 |
| Fig. 10 a | Krankheitstextur und -form für Morbus Bechterew, anhand von vier Patienten in unterschiedlichen Krankheitsstadien: 1. entzündliches Stadium mit |

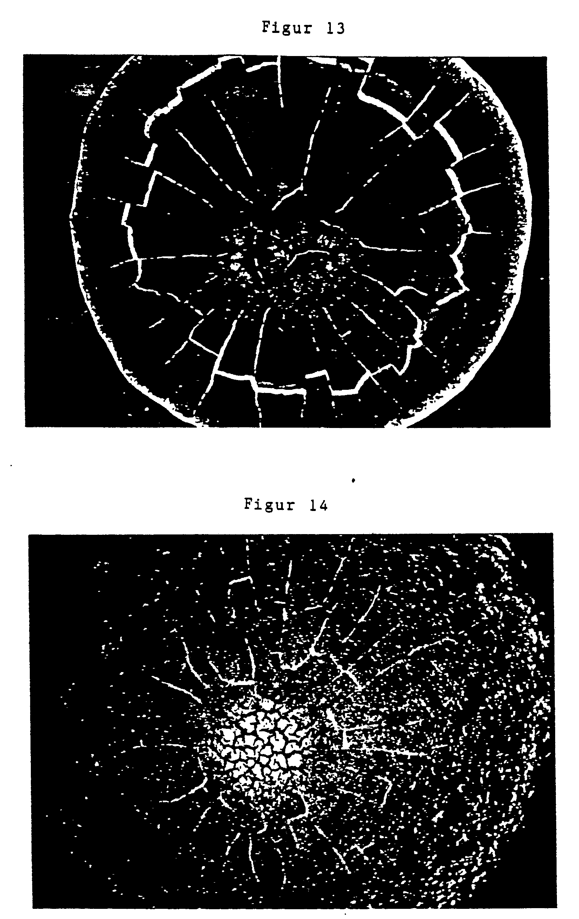

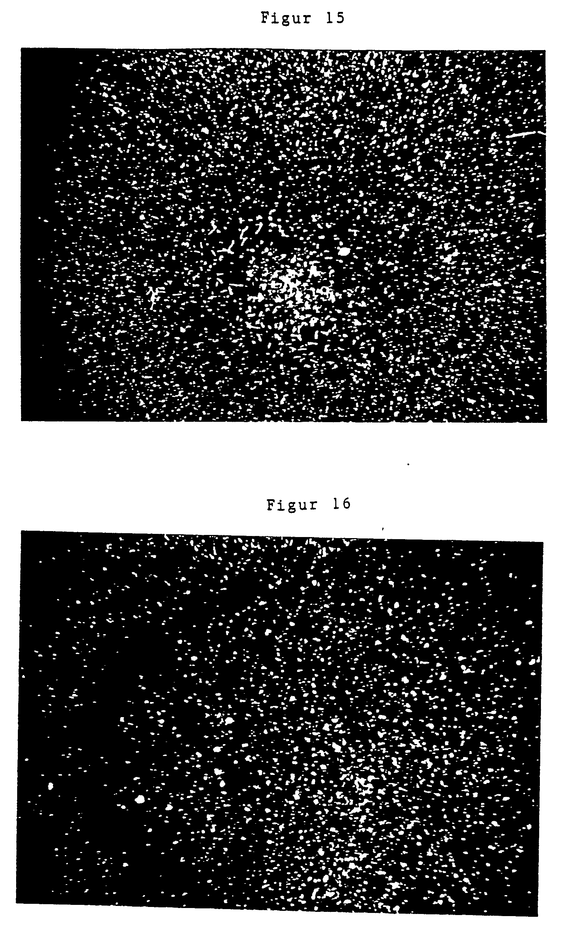

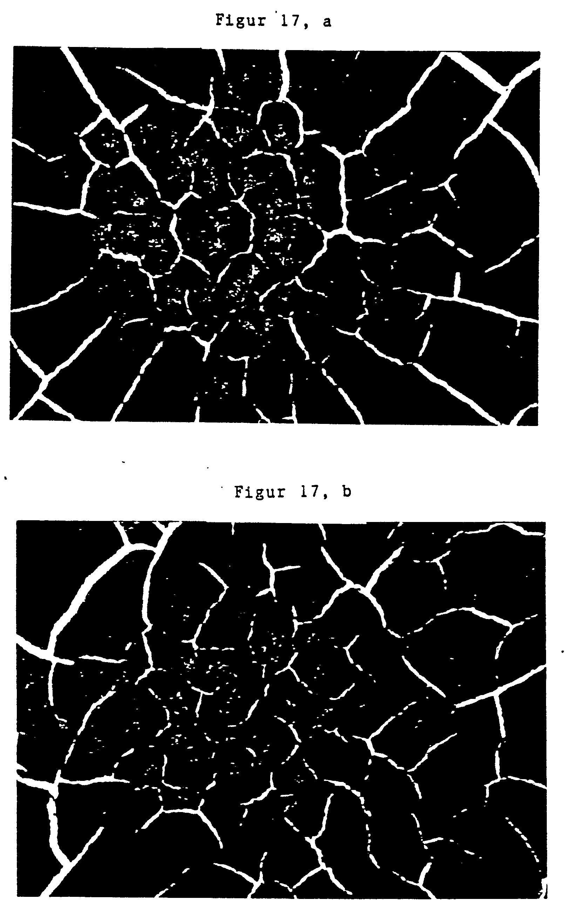

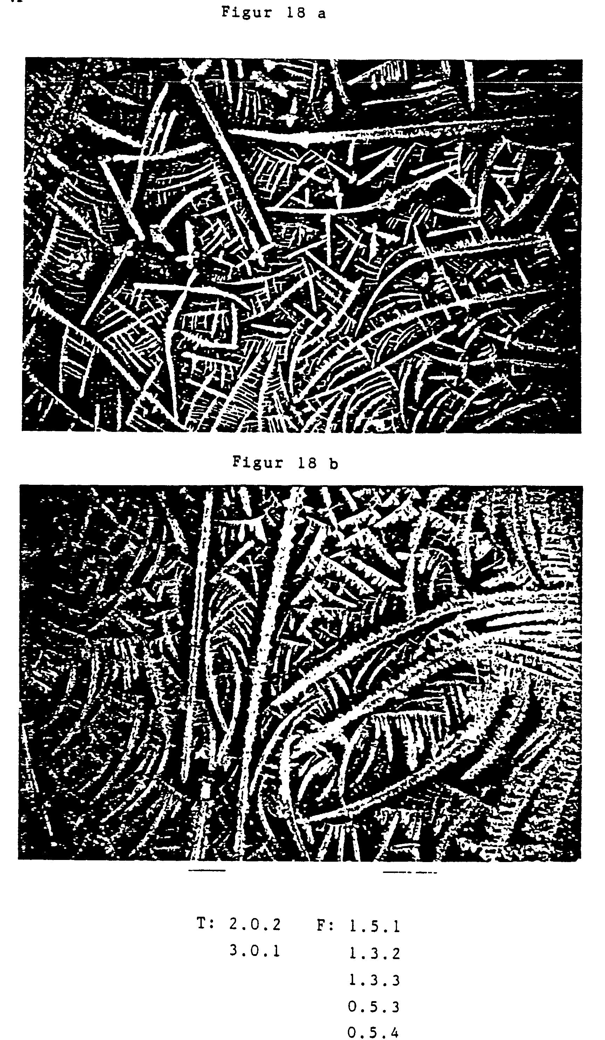

Fig. 10 b 2. Lähmungsgefühl
Versteifung der Halswirbelsäule
Fig. 10 c 3. Versteifung der Wirbelsäule bei
eingeschränkter
Bewegungsfähigkeit des Kopfes;:
Fig. 10 d 4. Völlige Unbeweglichkeit der
Wirbelsäule, keine Bewegung des
Kopfes mehr möglich.
Alle Typen: F: 1.9.3
Fig. 11 a Analogkristalle zu der
Krankheitsserie Fig. 10 a - d
Arnica montana, Bergwohlverleih,
F: 1.9.3
Fig. 11 b Gratiola officinalis,
Gottesgnadenkraut, F: 1.9.3
Fig. 11 c Aconitum napellus, Echter
Sturmhut, F: 1.9.3
Fig. 12, a Richtung der Zugspannung in der
flüssigen Phase
Fig. 12, b Maximum der Abdampfung der
flüssigen Phase aus dem Tropfen
Fig.12, c Fliessrichtung der Stoffpartikel in
der flüssigen Phase
Fig. 12, d Maximum der Anhäufung der
Stoffpartikel in der Mittelzone des
Kristallfeldes
Fig. 13 Vollblut, Tropfen auf Objektträger
aufgetrocknet und bei 20° C und
56 % relativer Luftfeuchte in 18
min getrocket
Fig. 14 Vollblut wie Figur 13, jedoch mit
tridestilliertem Wasser im
Verhältnis 1 : 1 vermischt
Fig. 15 Vollblut wie Figur 13, jedoch mit
tridestilliertem Wasser im
Verhältnis 1 : 9 vermischt
Fig. 16 Vollblut wie Figur 13, jedoch mit
tridestilliertem Wasser im
Verhältnis 1 : 39 vermischt
Fig.17, a, b Zentralfelder aus dem Vollblut
von 2 anderen Personen nach der
Behandlung wie nach Figur 13
Figur 18 a, b Kristallisat aus Vollblut wie Figur
13 nach dem Verfahren der
Erfindung.
T: 2.0.2. 3.0.1
F: 1.3.2. 1.3.3 1.5.1 0.5.3. 0.5.4

Es könnte die Frage entstehen, ob das Verfahren der Erfindung erforderlich ist, um ausgewählte, kennzeichnende, charakteristische, reproduzierbare Kristallbilder in Form von Kristalltexturen und Kristallformen zu erzeugen.

Es entsteht also die Frage, ob diese ausgewählten Kristallbilderin Form von Kristalltexturen und Kristallformen auch direkt aus dem Vollblut erzeugt werden können.

Die Kristallbilder der Figuren 13 bis 16 bestätigen in Abhängigkeit vom Verdünnungsgrad, ausgehend vom unverdünnten Vollblut bis zur 39-fachen Verdünnung mit tridestilliertem Wasser, dass die ausgewählten Kristalltexturen und Kristallformen nicht direkt aus dem Vollblut erzeugt werden können.

Die Figuren 17, a, b demonstrieren, dass aus dem Vollblut von anderen Personen anderen Alters nur die gleichen, reproduzierbaren Kristallbilder wie nach Figur 13 erzeugt werden, die keine Auswertung im Analogievergleich gemäss dem Verfahren der Erfindung gestatten.

Figur 18 demonstriert für das Vollblut der Figuren 13 - 16, dass nach dem Verfahren der Erfindung Kristalltexturen und Kristallformen differentierbar erzeugt werden.

Das Verfahren der Erfindung bietet den Vorteil der Frühdiagnose von Krankheitsbildern im Status nascendi und vor der diagnostischen Erkennung mit analytischen Verfahren nach dem Stand der Wissenschaft und Technik.

Das Verfahren der Erfindung bietet die Möglichkeit, das Prinzip des Analogievergleiches auf weitere Gebiete zur Kennzeichnung und Erfassung von Stoffen, oder von deren Spuren in anderen Substanzen durch Vergleich mit definierten Standarttypen der Kristalltexturen und Kristallformen anzuwenden.

Das Verfahren der Erfindung bietet weiter die Möglicheit, dem Krankheitsbild optimal angepasste Heilmittel aus definiertem, anorganischen, oder organischen, oder biologischem Material herzustellen. Das Verfahren der Erfindung ist mit technisch einfachen Verfahrensstufen bei geringem Investierungsaufwand mit hoher Sicherheit der Aussage und mit Reproduzierbarkeit bei der Wiederholung durchzuführen.

Dem Verfahren der Erfindung kommt somit eine erhebliche volkswirtschaftliche und medizinische, wie technische Bedeutung zu.

**Patentansprüche**

1. Verfahren unter Einsatz von organischem, und/oder biologischem, und/oder anorganischem Material zur Herstellung von Kristallbildern zur Feststellung von physiologischen Anomalien, oder zur Erkennung von Krankheiten, oder zur Kennzeichnung von Stoffen durch und zur Herstellung von Heilmitteln durch optischen Analogievergleich zu in gleicher Weise erzeugten, charakteristischen Typen solcher Kristallbilder, wobei aus der flüssigen, insbesondere wässrigen Phase von menschlichem venösem oder arteriellem Vollblut oder von Sekreten oder Ausscheidungen oder von Gewebeteilen von Mensch oder Tier, oder aus zerkleinerten, vergorenen Pflanzen oder Planzenteilen ein Destillat, gegebenenfalls bei 2,6 bis 3,2 bar, und aus dem zwischen 90 bis 140° C getrockneten, resultierenden Rückstand ein Kalzinat in der Weise erzeugt wird, dass die Temperatur bis auf 450° C bei Rauchgasentwicklung und teilweisem Verglimmen unter Nachziehen von Luft gesteigert und danach bis auf 550° C unter kurzzeitiger Flammentwicklung mit verstärkter Rauchgasentwicklung gesteigert und danach bis auf 750° C bis die Entgasung und bis das Nachziehen von Luft zum Stillstand kommt und bis das Kalzinat durchgeglüht ist, gesteigert, und danach die Temperatur auf bis etwa 1150°C unter Abdecken des Kalzinates zur Ausschaltung der Konvektionswärme bis zur Sinterung des Kalzinates gesteigert wird, und das Kalzinat nach dem Abkühlen mit dem Destillat zu einer Lösung oder Dispersion vermischt wird, und das Stoffgemisch in Form von einigen Tropfen auf einen mikroskopischen Objektträger aufgebracht wird und in 15 bis 50 min bei 17 bis 28°C bei einer relativen Luftfeuchtigkeit von 40 bis 80 %, oder in etwa 10 min bei etwa 90°C bei einer relativen Luftfeuchtigkeit von 60 bis 70 %, zur Bildung und zum mikroskopischen und photooptischen Analogievergleich von reproduzierbaren Kristallisaten als flächendeckende, typenbildende Kristallbilder mit Kristalltexturen und Kristallformen mit formtypischen und/oder diffusen, abgegrenzten Randzonen und Mittelzonen, oder mit Leerfeldern in der Kristallisationsfläche gebracht wird.

2. Verfahren nach Anspruch 1, worin die Kristalltexturen folgende, reproduzierbare, typische Figuren aufweisen:

Ausgehend von dem oberen, linken Punkt (0) befinden sich auf der Abszisse die Gruppen wie Typen und auf der Ordinate die Spezifikationen in den Typen: Abszisse:
(1)      bandförmig, parallel,
(2)      gitterförmig,
(3)      freie Lanzetten,
(4)      schlierenförmig,
(5)      rundförmig,
(1.0.1)  Parallele Bänder der Lanzetten,
(1.0.2)  regelmässig gewinkelte Bänder der Lanzetten,
(1.0.3)  versetzt gewinkelte Bänder der Lanzetten,
(1.0.4)  freie, winkelige Bandformen aus grossen und kleinen Lanzetten,
(1.0.5)  freie, winkelige Bandformen aus grossen Lanzetten,
(2.0.1)  rechtwinklige Gitter,
(2.0.2)  schiefwinklige Gitter,
(2.0.3)  rechtwinklige oder schiefwinklige Gitterbänder,
(2.0.4)  rechtwinklige oder schiefwinklige Gitterbänder mit gewellten Ästen oder Strichen,
(2.0.5)  bandförmige, gitterartige Rundformen,
(3.0.1)  gekreuzte oder freie Grosslanzetten,
(3.0.2)  freie, oder gekreuzte, mittellange Lanzetten,
(3.0.3)  freie, oder gekreuzte, kurze Lanzetten,
(3.0.4)  diffuses, flächendeckendes Konglomerat aus quasi- homogen verteilten Punktformen,
(3.0.5)  diffuses, nicht flächendeckendes, nicht homogenes Konglomerat von quasi-homogen verteilten Punktformen,
(4.0.1)  frei bewegliche, oder gekreuzte Schlierenform mit scharfen Konturen,

(4.0.2)  frei bewegliche, kleinere, oder gekreuzte Schlierenform mit scharfen Konturen,
(4.0.3)  frei bewegliche, oder gekreuzte Schlierenform mit unscharfen Konturen,
(4.0.4)  frei bewegliche Kleinschlieren,
(4.0.5)  haufenförmige Kleinstschlieren,
(5.0.1)  negative, haufenartige Rundformen
(5.0.2)  haufenförmige Rundformen,
(5.0.3)  flächendeckende Rundformen,
(5.0.4)  haufenförmige Punktformen.

3. Verfahren nach den Ansprüchen 1 oder 2, worin die Kristallformen folgende reproduzierbare, typische Figuren aufweisen: Ausgehend von dem oberen, linken Nullpunkt (0) eines Koordinatensystems, befinden sich auf der positiven Abszisse (0.1 - 0.9) folgende Kristallformen als Rundformen:
(0.1.1)  Punkt,
(0.2.1)  kleine, lichtmikroskopisch in der Flächentextur nicht differentierbare Kreise;

in der Flächetextur lichtmikroskopisch diffentierbare Formen:
(0.3.1)  differentierbare Kreise,
(0.4.1)  eliptoide Ovale,
(0.5.1)  gestreckte, eliptoide lanzettenförmige Formen, mit $F_1 F_2 = 2 (S_1 S_2)$
(0.6.1)  oval- nierenförmige Formen,
(0.7.1)  symmetrische, bauchige, gestreckte Lanzetten, mit $B_1 B_2 = 2 (S_1 S_2)$,
(0.8.1)  asymmetrische, bänderförmige, ungleichmässige Lanzetten,
(0.9.1)  freie amöben- ähnliche Formen mit prägnanten Einbuchtungen, $S_1 S2 \geq 2$ b,

und befinden sich auf der negativen Ordinate (1.1 - 1.9) folgende Kristallformen:
(1.1.1)  Punkte,
(1.2.1)  kurze Striche,
(1.3.1)  längere Striche,
(1.4.1)  zweischenklige Winkel von 10° bis 170°,
(1.5.1)  einfache kreuzförmige Winkel von 10° bis 90°,
(1.6.1)  geöffnete Dreiecke,
(1.7.1)  geschlossene Dreiecke,
(1.8.1)  Rauten und Quadrate,
(1.9.1)  Vielecke mit 5 und mehr Ecken,

und aus den Kristallformen (0.1 - 0.9) der Abszisse abgeleitet, befinden sich folgende Mischformen in Richtung der negativen Ordinate:
(0.3.2)  Kreise mit konzentrischen Innenkreisen,
(0.4.2)  gestreckte Ellipse mit stumpfen Enden mit $F_1 F_2 \leq S_1 S_2$,
(0.4.3)  stärker gestreckte Ellipse mit stumpfen Ende mit $F_1 F_2 > S_1 S_2$,
(0.5.2)  stark gestreckte, elliptoide Form mit

(0.5.3) anzetten-förmig mit stumpfen Enden und geringer Bauchung mit $F_1F_2 > 10$ $(S_1S_2)$,

(0.5.4) langezogene Lanzette mit Stumpfen Enden ohne Bauchigkeit mit $F_1F_2 > 25$ $(S_1S_2)$.

(0.6.2) nierenförmig mit stärkeren konkaven Einbuchtungen,

(0.6.3) hörnchenförmig mit 2 parallelen Bögen bis zum Halbkreis,

(0.6.4.) offene, doppellinige Kreisform mit an einem Ende angeschlossenem Strich oder Bogen,

(0.6.5) offene oder geschlossene Acht,

(0.7.2) längsgestreckte, bauige Lanzetten mit spitzen Enden mit $B_1B_2 > 6$ $(S_1S_2)$,

(0.7.3) längere, gestreckte, bauchige Lanzetten mit spitzen Enden mit $B_1B_2 > 10$ $(S_1S_2)$,

(0.7.4) gestreckte Sinus-Form mit $0 < a < \frac{\pi}{4}$,

(0.7.5) linienförmige Halbkreise mit an einem Ende anschliessende, sinusförmige Linie,

(0.7.6) offene, kreisförmige Linie mit an einem offenen Ende anschliessender, sinusförmiger Linie,

(0.8.2) halbkreisartige Rundform mit zackenförmigen oder wellenförmigen Ausprägungen der Sekanten,

(0.8.3.) zerklüftete, kammförmige, einseitige oder zweiseitige Längsform,

(0.8.4) unregelmässige, an den Rändern aufgeraute Lanzettenform,

(0.8.5) unregelmässige, einseitige oder beidseitige, sägeartige Form,

(0.8.6) kammförmig, mit hohen, senkrechten Zinken mit wellenförmigen oder zahnförmigen, unregelmässigen Rändern,

(0.8.7) kammförmig mit hohen schräggestellten Zinken mit wellenförmigen oder zahnförmigen, unregelmässigen Rändern,

(0.9.2) amöbenhafte Rundform mit unregelmässigen $S_1S_2 > 3b$-Einbuchtungen,

(0.9.3) rundförmig mit unregelmässig geformten Einbuchtungen,

(0.9.4) rundförmig mit zwei sich gegenüber liegenden Einbuchtungen,

(0.9.5) rautenförmig mit abgerundeten Ecken,

(0.9.6) dreiecksförmig mit abgerundeten Ecken,

(0.9.7) flache Dreiecksform mit abgerundeten Ecken,

und von den Kristallformen der negativen Ordinate abgeleitet, befinden sich folgende Strichformen in Richtung der positiven Abszisse:

(1.4.2) mehrschenklige Büschelform mit drei bis neun Schenkeln,

(1.4.3) mehrschenklige Büschelform mit mehr als zehn Schenkeln,

(1.4.4) mehrschenklige Büschelform mit nach aussen oder innen gebogenen, zwei oder mehr Schenkeln,

(1.5.2) gekreuzte, zwei und mehr Strichformen durch einen Mittelpunkt,

(1.5.3) gekreuzte, nach aussen oder innen gebogene Strichformen durch einen Mittelpunkt,

(1.5.4) Strichformen mit nach aussen oder innen gerichteten Büscheln an beiden Enden,

(1.5.5) Strichformen mit von einem Ende ausgehenden zwei und mehr geraden oder gebogenen Schenkeln,

(1.6.2) offene Dreiecksform mit einer verlängerten Seite,

(1.6.3) scherenförmige Gitterform,

(1.6.4) Trapezform mit aufgesetzter Dreiecksform,

(1.6.5) Dreiecksform mit aufgesetzter, längerer Dreiecksform,

(1.6.6) Dreiecksform mit konkaver Drittseite,

(1.7.2) Dreiecksform mit zentralen Innenstrahlen aus einer Ecke,

(1.7.3) Dreiecksform mit zwei oder drei konkaven Seiten,

(1.7.4) Dreiecksform, keilförmig,

(1.7.5) Dreiecksform mit wellenförmigen Seiten und ein bis zwei abgeschnittenen Ecken,

(1.7.6) Trapezform,

(1.7.7) überlappende Dreiecksform,

(1.8.2) Quadratform,

(1.8.3) Quadratform mit konkaven Seiten,

(1.8.4) Quadratform mit ein bis drei eingeschnittenen Ecken,

(1.8.5) Quadratform mit ähnlicher Innenform,

(1.8.6) Quadratform mit runden Anlagerungen an den Ecken,

(1.8.7) Quadratform mit eckigen Anlagerungen an den Ecken,

(1.8.8) Quadratform mit punktförmigen oder haufenförmigen Anlagerungen an den Seiten,

(1.9.2) gestreckte Sechseckform,

(1.9.3) Rechteckform,

(1.9.4) zwei oder mehrere seitenparallele überlappte Rechteckformen,

(1.9.5) Rechteckform mit Abgrätschungen,

(1.9.6) geschieferte Rechteckform mit zwei gegenüberliegenden geraden und zwei unregelmässig gezackten oder gewellten Seiten,

(1.9.7) zwei und mehrfach überlappte nicht seitenparallele Rechteckformen,

(1.9.8) Rechteckform mit an beiden Stirnseiten aufgesetzten Dreieckformen,

(1.9.9) Doppeltrapezform.

4. Verfahren nach Anspruch 1, bei dem die Kalzinierung bei einer Temperatur bis etwa 450°C erfolgt zur Erzielung eines höheren Anteiles an grossflächigen Texturen mit geringerer Formendetaillierung, oder die Kalzination bei einer Temperatur bis etwa 750°C erfolgt zur Erzielung eines höheren Anteiles an Einzelformen gegenüber den Kristalltexturen zur Verstärkung der Aussagedeutlichkeit der Einzelformen.

5. Verfahren nach Anspruch 1, bei dem Kristallisate von Wasser, insbesondere Mineralwasser, in Abhängigkeit von der Zeit gegenüber dem Ausgangszustand zur Ermittlung von mit anderen analytischen Methoden nicht messbaren strukturellen und texturellen Veränderungen untersucht und verglichen werden, oder dass Kristallisate aus unbekannten Ausgangsstoffen, insbesondere von Pflanzen und Pflanzenteilen, im Analogievergleich zu einer Vielzahl von aus Serienversuchen gewonnenen, definierten Kristallbildern, deren Ausgangsstoffe bekannt sind, mit ähnlichen Kristalltexturen und Kristallformen untersucht werden, oder dass Kristallisate von Nahrungsmitteln zur Ermittlung auf pathogene, insbesondere auf karzinome Inhaltsstoffe, im Vergleich zu Kristallisaten aus definierten, frischen Nahrungsmitteln untersucht werden, oder dass Substanzen auf toxische Schwermetalle im Veigleich zu aus einer Vielzahl von Vergleichsserien gewonnenen Kristallbildern von Schwermetallen untersucht werden, wobei die Kristallisate jeweils in gleicher Weise aus dem Ausgangsstoff und dem zu prüfenden Stoff erzeugt werden.

6. Verfahren nach Anspruch 1, bei dem das Destillat dadurch erzeugt wird, dass nicht kristallisierende anorganische oder organische Stoffe mit der etwa 3 bis 4- fachen Menge mehrfach destilliertem Wasser mehrere, insbesondere 3 bis 7 Tage, einer Rückflussdestillation unterworfen werden und danach die flüssige Phase überdestilliert wird, und das Destillat ohne ein Kalzinat auf einen mikrooptischen Objektträger aufgetropft, und das erzeugte Kristallbild zur Herstellung von reproduzierbaren Kristalltexturen und Kristallformen, zum Analogievergleich verwendet wird.

7. Verfahren nach Anspruch 1, worin zu dem durch Analogievergleich frühdiagnostizierten Krankheitsbild ein Heilmittel zur Therapie dadurch hergestellt wird, dass aus organischen Stoffen, insbesondere aus Pflanzen oder Pflanzenteilen, oder aus Gewebe von Mensch oder Tier aus dem Destillat und dem Kalzinat möglichst gleichartige Kristalltexturen und Kristallformen ermittelt werden und aus diesem organischem Material in gleicher Weise eine homogene, wässrige Lösung aus Destillat und Kalzinat als Urtinktur hergestellt wird.

8. Verfahren nach Anspruch 1, worin bei Verwendung von venösem oder arteriellem Vollblut oder von Sekret, eine flächenhafte Aufteilung der Kristalltexturen und der Kristallformen durch die selektive Fliessgeschwindigkeit (mm/sec) der Stoffkomponenten des Tropfens bis zu deren Fixierung durch die Kristallisation und damit eine Zuordnung zu den definierten, menschlichen Organen oder Funktionskreisen erfolgt, derart, dass dem entodermen Bereich (A) eine bevorzugte Teilfläche in radialer Richtung im äusseren, konzentrischem Ringraum, und dem mesodermen Bereich (B) eine bevorzugte Teilfläche in radialer Richtung im mittleren, konzentrischen Ringraum und dem ektodermen Bereich (C) der zentrale Ringraum zugeordnet ist.

9. Verfahren nach Anspruch 8, worin dem
entodermen Bereich (A),
mesodermen Bereich (B),
ektodermen Bereich (C)
hauptsächlich folgende Typen im Kristallisatfeld zugeordnet sind:
(A)    Kristalltextur: 1.0.1; 1.0.2: 3.0.4; 5.0.1
       Kristallformen: 0.3; 0.4; 0.5; 0.7
(B)    Kristalltextur: 4.0.1,
       Kristallformen: 1.7: 1.8, 1.9; 0.8; 0.9,
(C)    Kristalltextur: 1.0.2; 1.0.3; 2.0.3; 5.0.3,
       Kristallformen: 1.3; 1.5.

10. Verfahren nach Anspruch 1, bei dem der Analogievergleich aus, von einander räumlich und/ oder zeitlich unabhängig erzeugten Kristallisaten durch mehrfache Ermittlung gleicher oder gleichförmiger Kristalltexturen und Kristallformen durch mikroskopische oder fotooptische Betrachtung erfolgt, oder durch mathematische Auswertung von Rasterfeldern der Kristallbilder oder durch computerisierte Auswertung von Programmen.

**Claims**

1. Process employing organic and/or biological and/or inorganic material for the production of crystal patterns for determining physiological anomalies or for diagnosing diseases or for marking substances and for the preparation of drugs by optical comparison to characteristic types of such crystal patterns which have been produced in the same manner, wherein a distillate is produced, if necessary under 2.6 to 3.2 bar, from the liquid, in particular aqueous phase of human venous or arterial whole blood or of secretions or excretions or of human or animal tissue parts, or from comminuted, fermented plants or plant parts, and a calcined material is produced from the resulting residue, dried at between 90 and 140°C, by a procedure in which the temperature is increased to 450°C with evolution of fumes and partial glowing and with intake of air, and is then increased to 550°C with brief production of flames and with more pronounced evolution of fumes, and is then increased to 750°C until degassing and the intake of air ceases and until the calcined material has been completely ignited, and thereafter the temperature is increased to about 1150°C, the calcined material being covered to exclude convectional heat, until sintering of the calcined material takes place, and the calcined material is cooled and then mixed with the distillate to give a solution or dispersion, and the mixture of substances is applied in the form of a few drops to a microscope slide and is brought, in 15 to 50 minutes at 17 to 28°C at a relative atmospheric humidity of 40 to 80 %, or in about 10 minutes at about 90°C at a relative atmospheric humidity of 60 to 70 %, for the formation and microscopic and photooptical comparison of reproducible crystals, into the form of continuous typical crystal patterns with crystal textures and crystal forms having typically shaped and/or diffuse, bounded edge zones and middle zones, or having empty spaces in the crystallization area.

2. Process according to Claim 1, wherein the crystal textures have the following, reproducible, typical figures: starting from the upper left point (0), the abscissa contains the groups in the form of types and the ordinate contains the specifications in the types:

Abscissa:

(1) Band-shaped, parallel,
(2) grid-like,
(3) free lancets,
(4) striated,
(5) circular,
(1.0.1) parallel bands of the lancets,
(1.0.2) regularly angled bands of the lancets,
(1.0.3) staggered angled bands of the lancets,
(1.0.4) free, angled band shapes comprising large and small lancets,
(1.0.5) free, angled band shapes comprising large lancets,
(2.0.1) right-angled grids,
(2.0.2) oblique-angled grids,
(2.0.3) right-angled or oblique-angled grid-like bands,
(2.0.4) right-angled or oblique-angled grid-like bands with wavy branches or lines,
(2.0.5) band-like, grid-like circular shapes,
(3.0.1) crossed or free lancets,
(3.0.2) free, or crossed, lancets of medium length,
(3.0.3) free, or crossed, short lancets,
(3.0.4) diffuse, continuous conglomerate comprising quasi-homogeneously distributed points,
(3.0.5) diffuse, non-continuous, nonhomogeneous conglomerate comprising quasi-homogeneously distributed points,
(4.0.1) freely mobile or crossed stria pattern with sharp contours,
(4.0.2) freely mobile, relatively small or crossed stria pattern with sharp contours,
(4.0.3) freely mobile or crossed stria pattern with diffuse contours,
(4.0.4) freely mobile small striae,
(4.0.5) very small agglomerated striae,
(5.0.1) negative, agglomerated circular shapes,
(5.0.2) agglomerated circular shapes,
(5.0.3) continuous pattern of circular shapes and

(5.0.4) agglomerated points.

3. Process according to Claim 1 or 2, wherein the crystal forms have the following reproducible, typical figures:

starting from the upper, left zero point (0) of a coordinate system, the following crystal forms are present as circular shapes along the positive abscissa (0.1 - 0.9):

(0.1.1) point,
(0.2.1) small circles whose surface texture is not distinguishable under the optical microscope;

shapes whose surface texture is distinguishable under the optical microscope:

(0.3.1) distinguishable circles,
(0.4.1) ellipsoidal ovals,
(0.5.1) elongated, ellipsoidal lancet-like shapes, with $F_1F_2 = 2 (S_1S_2)$,
(0.6.1) oval kidney-shaped forms,
(0.7.1) symmetrical, convex, elongated lancets, with $B_1B_2 = 2(S_1S_2)$
(0.8.1) asymmetrical, band-like, irregular lancets,
(0.9.1) free, amoeba-like shapes with sharp indentations, $S_1S_2 \geqslant 2b$,

and the following crystal forms are located on the negative ordinate (1.1 - 1.9):

(1.1.1) Points,
(1.2.1) short lines,
(1.3.1) longer lines,
(1.4.1) two-limbed angle of 10° to 170°,
(1.5.1) simple, cruciform angle of 10° to 90°
(1.6.1) open triangles,
(1.7.1) closed triangles,
(1.8.1) rhombuses and squares,
(1.9.1) polygons with 5 or more corners,

and, derived from the crystal forms (0.1 - 0.9) of the abscissa, the following mixed forms are located in the direction of the negative ordinate:

(0.3.2) circles with concentric inner circles,
(0.4.2) elongated ellipses with rounded ends, with $F_1F_2 \leqslant S_1S_2$,
(0.4.3) more elongated ellipses with rounded ends, with $F_1F_2 > S_1S_2$,
(0.5.2) very elongated, ellipsoidal shape with rounded ends, lancet-like with $F_1F_2 > 4(S_1S_2)$,
(0.5.3) lancet-like with rounded ends and slightly convex shape with $F_1F_2 > 10(S_1S_2)$,
(0.5.4) elongated lancet with rounded ends without convex shape, with $F_1F_2 > 25(S_1S_2)$,
(0.6.2) kidney-shaped with relatively pronounced concave indentations,
(0.6.3) crescent-shaped with two parallel curves forming a semicircle,
(0.6.4) open, double-lined circular shape with a line or curve connected to one end,
(0.6.5) open or closed figure of eight,
(0.7.2) elongated, convex lancets with pointed ends with $B_1B_2 > 6(S_1S_2)$,
(0.7.3) relatively long, elongated, convex lancets with pointed ends with $B_1 B_2 > 10(S_1 S_2)$,

(0.7.4) elongated sinusoidal shape with $0 < a < \frac{\pi}{4}$,

(0.7.5) linear semicircles with sinusoidal line connected to one end,

(0.7.6) open, circular line with sinusoidal line connected to an open end,

(0.8.2) semicircular shape with toothed or wavy secants,

(0.8.3) fissured, comb-like, one-sided or two-sided elongated shape,

(0.8.4) irregular lancet shape with rough edges,

(0.8.5) irregular, one-sided or two-sided saw-like shape,

(0.8.6) comb-like with high vertical prongs at right angles and with wavy or toothed, irregular edges,

(0.8.7) comb-like with high inclined prongs with wavy or toothed, irregular edges,

(0.9.2) amoeba-like circular shape with irregular indentations with $S_1 S_2 > 3b$,

(0.9.3) circular with irregularly shaped indentations,

(0.9.4) circular with two indentations opposite one another,

(0.9.5) rhombic with rounded corners,

(0.9.6) triangular with rounded corners,

(0.9.7) flattened triangular shape with rounded corners,

and, derived from the crystal forms of the negative ordinate, the following line patterns are located in the direction of the positive abscissa:

(1.4.2) Multi-limbed cluster with three to nine limbs,

(1.4.3) multi-limbed cluster with more than ten limbs,

(1.4.4) multi-limbed cluster with two or more limbs bent outwards or inwards,

(1.5.2) two or more lines intersecting at a midpoint,

(1.5.3) lines bent outwards or inwards and intersecting at a midpoint,

(1.5.4) lines with outward or inward pointing clusters at both ends,

(1.5.5) lines with two or more straight or bent limbs extending from one end,

(1.6.2) open triangular shape with one extended side,

(1.6.3) scissor-like grid pattern,

(1.6.4) trapezoid with attached triangle,

(1.6.5) triangle with attached, longer triangle,

(1.6.6) triangle with concave third side,

(1.7.2) triangle with central inner rays from one corner,

(1.7.3) triangle with two or three concave sides,

(1.7.4) triangle, wedge-shaped,

(1.7.5) triangle with wavy sides and one or two cut-off corners,

(1.7.6) trapezium,

(1.7.7) overlapping triangular shape,

(1.8.2) square,

(1.8.3) square with concave sides,

(1.8.4) square with one to three cut-end corners,

(1.8.5) square with similar inner shape,

(1.8.6) square with round shapes attached to the corners,

(1.8.7) square with angular shapes attached to the corners,

(1.8.8) square with point-like or agglomerate-like deposits on the sides,

(1.9.2) elongated hexagonal shape,

(1.9.3) rectangle,

(1.9.4) two or more overlapped rectangles with parallel sides,

(1.9.5) rectangle with scissor-like projections,

(1.9.6) foliated rectangular shape with two straight sides opposite one another and two irregularly toothed or wavy sides,

(1.9.7) doubly or multiply overlapped oblong shapes without parallel sides,

(1.9.8) rectangle with triangles attached to both ends and

(1.9.9) double trapezium.

4. Process according to Claim 1, wherein the calcination is carried out at a temperature of up to about 450°C for achieving a higher proportion of textures having a large area and less shape detail, or the calcination is carried out at a temperature of up to about 750°C to achieve a higher proportion of individual shapes compared with the crystal textures, to ensure that the individual shapes provide clearer information.

5. Process according to Claim 1, wherein crystals obtained from water, in particular mineral water, are investigated and compared with the initial state as a function of time in order to determine structural and textural changes which cannot be measured by other analytical methods, or that crystals of unknown starting materials, in particular of plants and plant parts, are investigated in comparison with a large number of defined crystal patterns which have been obtained from series experiments and whose starting materials are known and have similar-crystal textures and crystal forms, or that crystals obtained from foods are investigated in comparison with crystals obtained from defined, fresh foods in order to determine pathogenic, in particular carcinomic ingredients, or that substances are investigated for toxic heavy metals in comparison with crystal patterns, of heavy metals, which have been obtained from a large number of comparative series, the crystals being produced each time in the same way from the starting material and from the material to be tested.

6. Process according to Claim 1, wherein the distillate is produced by subjecting noncrystalline inorganic or organic substances to reflux distillation with about 3 to 4 times the amount of multiply distilled water for several, in particular 3 to 7, days and then distilling over the liquid phase, and then introducing the distillate dropwise, without calcined material, on to a microscope slide and carrying out the comparison using the resulting crystal pattern for the production of reproducible crystal textures and crystal forms.

7. Process according to Claim 1, wherein, for the clinical picture diagnosed at an early stage by comparison, a therapeutic agent is prepared by a method in which as far as possible similar crystal textures and crystal forms are determined from organic substances, in particular from plants or plant parts, or from human or animal tissue from the distillate and the calcined material, and a homogeneous, aqueous solution of distillate and calcined material is prepared, as a primary tincture, from this organic material in the same manner.

8. Process according to Claim 1, wherein, when venous or arterial whole blood or secretion is used, the crystal textures and the crystal forms are distributed over a large area by the selective flow rate (mm/sec) of the components of the drop until the said components are fixed by the crystallization and are thus assigned to the defined, human organs or functional circles, in such a way that a preferred part area in the radial direction in the outer, concentric annular space is associated with the entodermal region (A), and a preferred part area in the radial direction in the middle, concentric annular space is associated with the mesodermal region (B), and the central annular space is associated with the ectodermal region (C).

9. Process according to Claim 8, wherein mainly the following types in the crystal area are associated with the
entodermal region (A),
mesodermal region (B) and
ectodermal region (C):

(A)     Crystal texture: 1.0.1; 1.0.2; 3.0.4; 5.0.1
        Crystal forms: 0.3; 0.4; 0.5; 0.7
(B)     Crystal texture: 4.0.1
        Crystal forms: 1.7; 1.8; 1.9; 0.8; 0.9
(C)     Crystal texture: 1.0.2; 1.0.3; 2.0.3; 5.0.3
        Crystal forms: 1.3; 1.5.

10. Process according to Claim 1, wherein the comparison is carried out for crystals produced at different sites and/or different times, by determining identical or similar crystal textures and crystal forms several times by microscopic or photo-optical inspection, or by mathematical evaluation of screens of the crystal patterns or by computerized evaluation by programs.

**Revendications**

1. Procédé dans lequel on utilise une matière organique et/ou biologique et/ou inorganique en vue de former des cristallogrammes pour la détermination d'anomalies physiologiques, ou pour déceler des maladies, ou encore pour caractériser des substances et pour préparer des remèdes par comparaison analogique optique avec des types caractéristiques (obtenus de la même manière) de ces cristallogrammes, procédé dans lequel, à partir de la phase liquide, en particulier, de la phase aqueuse du sang complet veineux ou artériel humain ou de sécrétions ou encore de précipitations ou de parties de tissus de l'homme ou de l'animal, ou encore à partir de plantes ou de parties de plantes broyées et fermentées, on prépare un distillat, éventuellement sous une pression de 2,6 à 3,2 bars et, à partir du résidu obtenu séché à une température comprise entre 90 et 140°C, on prépare un produit de calcination en portant la température à 450°C avec un dégagement de gaz de fumée et en consumant partiellement peu à peu avec extraction de l'air tandis que, ensuite, on porte cette température à 550°C avec dégagement de flammes pendant une courte période et avec dégagement renforcé de gaz de fumée, après quoi, on la porte à 750°C jusqu'à dégazage et jusqu'à extraction de l'air pour aboutir à l'arrêt et jusqu'à ce que le produit de calcination soit complètement calciné, puis on porte la température à environ 1.150°C en recouvrant le produit de calcination pour exclure la chaleur de convection jusqu'au frittage du produit de calcination et, après refroidissement, on mélange le produit de calcination avec le distillat pour former une solution ou une dispersion et l'on applique le mélange de matières sous forme de quelques gouttes sur un porte-objet microscopique et, au cours d'une période de 15 à 50 minutes, on l'amène à une température de 17 à 28°C à une humidité atmosphérique relative de 40 à 80 % ou, au cours d'une période d'environ 10 minutes, à une température d'environ 90°C à une humidité atmosphérique relative de 60 à 70 %, en vue de former et d'obtenir une comparaison analogique microscopique et photooptique de cristallisats reproductibles sous forme de cristallogrammes à couverture superficielle formant des caractères avec des textures et des formes cristallines comportant des zones marginales et des zones centrales limitées de forme typique et/ou diffuses, ou avec des champs à vide dans la surface de cristallisation.

2. Procédé selon la revendication 1, dans lequel les textures cristallines ont les figures spécifiques reproductibles suivantes:

Partant du point supérieur gauche (0), sur l'abscisse, se trouvent les groupes tels que les types et, sur l'ordonnée, les spécifications dans les types:

Abscisse:
(1)     sous forme de bandes parallèles,
(2)     sous forme d'une grille,
(3)     lancettes libres,
(4)     sous forme de stries,
(5)     sous forme circulaire,
(1.0.1) bandes parallèles des lancettes,
(1.0.2) bandes régulièrement coudées des lancettes,
(1.0.3) bandes coudées avec décalage, des lancettes,
(1.0.4) formes de bandes coudées libres des grandes et des petites lancettes,
(1.0.5) formes de bandes coudées libres des grandes lancettes,
(2.0.1) grille à angle droit,
(2.0.2) grille à angle scalène,
(2.0.3) bandes de grilles à angle droit ou à angle scalène,
(2.0.4) bandes de grilles à angle droit ou à angle scalène avec des branches ou lignes ondulées,
(2.0.5) formes circulaires de type en grille et en forme de bandes,
(3.0.1) grandes lancettes croisées ou libres,
(3.0.2) lancettes libres ou croisées de longueur moyenne,
(3.0.3) lancettes courtes libres ou croisées,
(3.0.4) conglomérat diffus de couverture superficielle constitué de formes ponctuelles réparties de manière quasi homogène,
(3.0.5) conglomérat diffus sans couverture superficielle et non homogène de formes ponctuelles réparties de manière quasi homogène,
(4.0.1) forme striée pouvant se déplacer librement ou croisée comportant des contours prononcés,
(4.0.2) forme striée pouvant se déplacer librement, plus petite ou croisée avec des contours prononcés,
(4.0.3) forme striée pouvant se déplacer librement ou croisée avec des contours non prononcés,
(4.0.4) petites stries pouvant se déplacer librement,
(4.0.5) petites stries sous forme de tas,
(5.0.1) formes circulaires négatives en forme de tas,
(5.0.2) formes circulaires en forme de tas,
(5.0.3) formes circulaires à recouvrement superficiel,
(5.0.4) formes ponctuelles en forme de tas.

3. Procédé selon les revendications 1 ou 2, caractérisé en ce que les formes cristallines possèdent les figures spécifiques reproductibles suivantes:

Partant du point zéro supérieur gauche (0) d'un système de coordonnées, sur l'abscisse positive (0,1 - 0,9), se trouvent les formes cristallines suivantes sous des formes circulaires:

(0.1.1) point,
(0.2.1) petits cercles ne pouvant être différenciés dans leur texture superficielle au microscope optique;

formes pouvant être différenciées dans leur texture superficielle au microscope optique:

(0.3.1) cercles pouvant être différenciés,
(0.4.1) ovales ellipsoïdes,
(0.5.1) formes de lancettes ellipsoides étirées, avec $F_1F_2 = 2 (S_1S_2)$,
(0.6.1) formes réniformes ovales,
(0.7.1) lancettes étirées bombées symétriques, avec $B_1B_2 = 2 (S_1S_2)$,

(0.8.1) lancettes irrégulières en forme de bandes asymétriques,
(0.9.1) formes libres analogues à des amibes avec dentelures significatives $S_1S_2 \geqq 2$ b,

et, sur l'ordonnée négative (1,1 - 1,9), se trouvent les formes cristallines suivantes:

(1.1.1) points,
(1.2.1) traits courts,
(1.3.1) traits plus longs,
(1.4.1) angle à deux branches de 10 à 170°,
(1.5.1) angle simple en croix de 10 à 90°,
(1.6.1) triangle ouvert,
(1.7.1) triangle fermé,
(1.8.1) losanges et carrés,
(1.9.1) polygones à cinq angles et plus,

et, dérivées des formes cristallines (0,1 - 0,9) de l'abscisse, se trouvent les formes mixtes suivantes dans le sens de l'ordonnée négative:

(0.3.2) cercles avec cercles intérieurs concentriques,
(0.4.2) ellipse allongée avec extrémités tronquées et $F_1F_2 \leqq S_1S_2$,
(0.4.3) ellipse plus fortement allongée avec extrémités tronquées et $F_1F_2 > S_1S_2$
(0.5.2) forme elliptique fortement allongée avec extrémités tronquées sous forme de lancettes avec $F_1F_2 > 4 (S_1S_2)$,
(0.5.3) forme de lancettes avec extrémités tronquées et ventre plus faible avec $F_1F_2 > 10 (S_1S_2)$
(0.5.4) lancette allongée avec extrémités tronquées sans ventre et $F_1F_2 > 25 (S_1S_2)$
(0.6.2) réniforme avec dentelures concaves plus fortes,
(0.6.3) forme de cornes avec deux arcs parallèles jusqu'au demi-cercle,
(0.6.4) forme de cercle ouvert à ligne double avec un trait ou un arc raccordé à une extrémité,
(0.6.5) octogone ouvert ou fermé,
(0.7.2) lancettes ventrues étirées dans le sens longitudinal avec extrémités pointues et $B_1B_2 > 6 (S_1S_2)$,
(0.7.3) lancettes ventrues étirées et plus longues avec des extrémités pointues et $B_1B_2 > 10 (S_1S_2)$,
(0.7.4) forme sinusoïdale étirée avec 0 < à < $\frac{\pi}{4}$,
(0.7.5) demi-cercle linéaire avec ligne sinusoïdale raccordée à une extrémité,
(0.7.6) ligne circulaire ouverte suivie, à une extrémité ouverte, d'une ligne sinusoïdale,
(0.8.2) forme circulaire en demi-cercle avec empreintes dentelées ou ondulées des sécantes,
(0.8.3) forme longitudinale unilatérale ou bilatérale en forme de peigne crevassée,
(0.8.4) forme de lancette irrégulière à bords rendus rugueux,
(0.8.5) forme de scie irrégulière, unilatérale ou bilatérale,

| | |
|---|---|
| (0.8.6) | en forme de peigne, avec de hautes dents verticales comportant des bords ondulés ou dentelés irréguliers, |
| (0.8.7) | en forme de peigne, avec de hautes dents en oblique comportant des bords ondulés ou dentelés irréguliers, |
| (0.9.2) | forme circulaire sous forme d'amibe avec des dentelures irrégulières $S_1 S_2 > 3b$ - |
| (0.9.3) | forme circulaire avec dentelures de forme irrégulière, |
| (0.9.4) | de forme circulaire avec deux dentelures se plaçant l'une contre l'autre, |
| (0.9.5) | en forme de losange avec des angles arrondis, |
| (0.9.6) | en forme de triangle avec des angles arrondis, |
| (0.9.7) | en forme de triangle plat avec des angles arrondis, |

et dérivant des formes cristallines des ordonnées négatives, se trouvent les formes de traits suivantes dans le sens de l'abscisse positive:

| | |
|---|---|
| (1.4.2) | forme de touffe à plusieurs branches comportant trois à neuf branches, |
| (1.4.3) | forme de touffe à plusieurs branches comportant plus de dix branches, |
| (1.4.4) | forme de touffe à plusieurs branches comportant deux branches ou plus pliées vers l'extérieur ou vers l'intérieur, |
| (1.5.2) | deux formes de traits et plus croisées passant par un point central, |
| (1.5.3) | formes de traits croisées pliées vers l'extérieur ou vers l'intérieur et passant par un point central, |
| (1.5.4) | formes de traits avec, aux deux extrémités, des touffes dirigées vers l'extérieur ou vers l'intérieur, |
| (1.5.5) | formes de traits avec deux branches et plus droites ou pliées partant d'une extrémité, |
| (1.6.2) | forme de triangle ouvert avec un côté allongé, |
| (1.6.3) | forme de grille en cisaille, |
| (1.6.4) | forme trapézoïdale surmontée d'une forme triangulaire, |
| (1.6.5) | forme triangulaire surmontée d'une forme triangulaire plus longue, |
| (1.6.6) | forme triangulaire avec un troisième côté concave, |
| (1.7.2) | forme triangulaire avec des rayons intérieurs centraux partant d'un angle, |
| (1.7.3) | forme triangulaire avec deux ou trois côtés concaves, |
| (1.7.4) | forme triangulaire, cunéiforme, |
| (1.7.5) | forme triangulaire avec côtés ondulés et un à deux angles coupés, |
| (1.7.6) | forme trapézoïdale, |
| (1 7 7) | forme triangulaire à chevauchement, |
| (1.8.2) | forme carrée, |
| (1.8.3) | forme carrée avec côtés concaves, |
| (1.8.4) | forme carrée avec un à trois angles entaillés, |
| (1.8.5) | forme carrée avec forme intérieure analogue, |
| (1.8.6) | forme carrée avec incrustations circulaires aux angles, |
| (1.8.7) | forme carrée avec incrustations angulaires aux angles, |
| (1.8.8) | forme carrée avec incrustations ponctuelles ou entassées sur les côtés, |
| (1.9.2) | forme hexagonale étirée, |
| (1.9.3) | forme rectangulaire, |
| (1.9.4) | deux ou plusieurs formes rectangulaires se chevauchant et à côtés parallèles, |
| (1.9.5) | forme rectangulaire avec ébarbages, |
| (1.9.6) | forme rectangulaire en oblique avec deux côtés droits opposés et deux côtés dentelés ou ondulés irrégulièrement, |
| (1.9.7) | formes rectangulaires à côtés non parallèles se chevauchant deux et plusieurs fois, |
| (1.9.8) | forme rectangulaire avec des formes triangulaires placées sur deux côtés frontaux, |
| (1.9.9) | forme trapézoïdale double. |

4. Procédé selon la revendication 1, dans lequel la calcination a lieu à une température allant jusqu'à environ 450° C afin d'obtenir une plus haute fraction de textures à grande surface avec une plus faible mise en détail des formes, ou en ce que la calcination a lieu à une température allant jusqu'à environ 750° C afin d'obtenir une plus haute fraction de formes individuelles vis-à-vis des textures cristallines en vue du renforcement de la netteté de prévision des formes individuelles.

5. Procédé selon la revendication 1, dans lequel les cristallisats d'eau, en particulier, d'eau minérale, sont examinés et comparés en fonction du temps vis-à-vis de l'état initial afin de déterminer des modifications de structures et de textures non mesurables par d'autres méthodes analytiques, ou en ce que les cristallisats sont examinés à partir de substances de départ inconnues, en particulier, de plantes et de parties de plantes, en une comparaison analogique avec un grand nombre de cristallogrammes définis obtenus par des essais en série, dont les substances de départ sont connues, avec des textures et des formes cristallines analogues, ou en ce que les cristallisats d'aliments sont examinés en vue de déterminer les constituants pathogènes, en particulier, les constituants carcinomes, comparativement à des cristallisats d'aliments frais définis, ou en ce que les substances sont examinées concernant les métaux lourds toxiques comparativement à plusieurs cristallogrammes de métaux lourds, obtenus par des séries de comparaisons, les cristallisats étant obtenus chaque fois de la même manière à partir de la substance de départ et de la substance à examiner.

6. Procédé selon la revendication 1, dans lequel le distillat est formé du fait que des substances organiques ou inorganiques ne cristallisant pas sont soumises avec de l'eau distillée à plusieurs reprises en une quantité de 3 à 4 fois, en particulier, pendant 3 à 7 jours, à une distillation à reflux, après quoi la phase liquide est soumise à une distillation et le distillat est appliqué goutte à goutte, sans produit de calcination, sur un porte-objet micro-optique et le cristallogramme obtenu est utilisé pour préparer des formes et des textures cristallines reproductibles en vue d'une comparaison analogique.

7. Procédé selon la revendication 1, dans lequel, d'après le bulletin de santé diagnostiqué prématurément par une comparaison analogique, on prépare un remède pour la thérapie en déterminant, d'après des substances organiques, en particulier, provenant de plantes ou de parties de plantes, ou encore de tissus humains ou animaux, à partir du distillat et du produit de calcination, des textures et des formes cristallines de nature aussi identique que possible tandis que, à partir de cette matière organique, on prépare, de la même manière, une solution aqueuse homogène constituée du distillat et du produit de calcination comme teinture de base.

8. Procédé selon la revendication 1, dans lequel, lors de l'utilisation de sang complet veineux ou artériel ou de sécrétions, on effectue une répartition superficielle des textures ou des formes cristallines par la vitesse d'écoulement sélective (mm/seconde) des composants des substances des gouttes jusqu'à leur fixation par la cristallisation et, partant, une attribution aux circuits fonctionnels ou aux organes humains définis de telle sorte qu'une surface partielle préférée soit attribuée à la zone entoderme (A) dans la direction radiale dans l'espace annulaire concentrique extérieur et qu'une surface partielle préférée soit attribuée à la zone mésoderme (B) dans une direction radiale dans le compartiment central concentrique, ainsi qu'à la zone ectoderme (C) de l'espace annulaire central.

9. Procédé selon la revendication 8, dans lequel à la zone entoderme (A),
à la zone mésoderme (B), et
à la zone ectoderme (C),
les types suivants sont principalement attribués dans le champ du produit de cristallisation:
(A)    Texture cristalline: 1.0.1; 1.0.2; 3.0.4; 5.0.1
       Formes cristallines: 0.3; 0.4; 0.5; 0.7,
(B)    Texture cristalline: 4.0.1
       Formes cristallines: 1.7; 1.8; 1.9; 0,8; 0.9,
(C)    Texture cristalline: 1.0.2; 1.0.3; 2.0.3; 5.0.3,
       Formes cristallines: 1.3; 1.5.

10. Procédé selon la revendication 1, dans lequel la comparaison analogique a lieu à partir de produits de cristallisation obtenus indépendamment l'un de l'autre dans l'espace et/ou dans le temps, par détermination multiple de textures et de formes cristallines identiques ou de même forme par examen microscopique ou photo-optique, ou par évaluation mathématique de champs de grilles de cristallogrammes ou par évaluation de programmes au moyen d'ordinateurs.

Figur 1

EP 0 236 294 B1

Figur 2

Figur 3

Figur 4,a

Figur 4,b

Figur 4,c

Figur 4,a:
T: 1.0.1;5.0.3
F: 1.7.4

Figur 4,b:
T: 2.0.5

Figur 4,c:
T: 2.0.5

7

Figur 5,a

Figur 5,b

Figur 5, c

Figur: 5,a ; 5, b; 5, c:

T: 5.0.1

F: 0.3.2

Figur 6,a

F: 0.8.6

Figur 6,b

F: 0.8.6

Figur 6,c

F: 0.8.6

Figur 7, a

Figur 7, b

Figur: 7,a; 7,b;

T: 2.0.1

F: 1.9.1

Figur 7, c

Figur 7, d

Figur: 7, c; 7, d:

T: 2.0.1

F: 1.9.1

17

Figur 8, a

F: 1.6.4

Figur 8, b

F: 1.7.4

Figur 9, a

F: 1.3.2
   0.1.1

Figur 9, b

F: 1.3.3
   1.5.1
   0.3.1

Figur 9, c

T: 2.0.3
F: 1.3.3

Figur 10, a

Figur 10, b

Figur: 10, a; 10, b:

F: 1.9.3

Figur 10, c

Figur 10, d

Figur: 10, c; 10, d:

F: 1.9.3

25

Figur 11, a

Figur 11, b

Figur 11, c

Figur 12

Figur 12,a

Figur 12,b

Figur 12,c

Figur 12,d

Figur 13

Figur 14

Figur 15

Figur 16

Figur 17, a

Figur 17, b

Figur 18 a

Figur 18 b

```
T: 2.0.2    F: 1.5.1
   3.0.1       1.3.2
               1.3.3
               0.5.3
               0.5.4
```